# EUROPEAN PATENT APPLICATION

(11) **EP 4 574 843 A1**
(43) Date of publication of application: **25.06.2025**
(21) Application number: 23220027.9
(22) Date of filing: 22.12.2023
(51) Int. Cl.: C07K 16/28, A61P 35/00, A61K 39/395

(54) **ANTIBODY BINDING DOMAINS HAVING SPECIFICITY FOR LILRB2**

(71) Applicant: Numab Therapeutics AG, 8810 Horgen (CH)
(72) Inventor: The designation of the inventor has not yet been filed
(74) Representative: Virnekäs, Bernhard

(57) **Abstract**

The present invention relates to an antibody binding domain, which specifically binds to LILRB2 (LILRB2-BD), to monospecific antibodies comprising two of said LILRB2-BDs and to multispecific antibodies comprising one or two of said LILRB2-BDs and at least one further binding domain, which specifically binds to a target different from LILRB2. The present invention further relates to nucleic acid sequences encoding said antibody binding domain, antibody or multispecific antibody, to vector(s) comprising said nucleic acid sequences, to host cell(s) comprising said nucleic acid sequences or said vector(s), and to a method of producing said multispecific antibody. Additionally, the present invention relates to pharmaceutical compositions comprising said antibody or multispecific antibody and methods of use thereof.

## Description

### FIELD OF THE INVENTION

The present invention relates to an antibody binding domain, which specifically binds to LILRB2 (LILRB2-BD), to monospecific antibodies comprising two of said LILRB2-BDs and to multispecific antibodies comprising one or two of said LILRB2-BDs and at least one further binding domain, which specifically binds to a target different from LILRB2. The present invention further relates to nucleic acid sequences encoding said antibody binding domain, antibody or multispecific antibody, to vector(s) comprising said nucleic acid sequences, to host cell(s) comprising said nucleic acid sequences or said vector(s), and to a method of producing said multispecific antibody. Additionally, the present invention relates to pharmaceutical compositions comprising said antibody or multispecific antibody and methods of use thereof.

### BACKGROUND OF THE INVENTION

LILRB2 belongs to the subfamily B class of LIR receptors and is expressed on immune cells, such as myeloid cells, including monocytes, macrophages, dendritic cells and granulocytes. It binds classical and non-classical major histocompatibility complex Class I (MHC-I) proteins on antigen-presenting cells, such as human leukocyte antigen A (HLA-A) and human leukocyte antigen G (HLA-G), and transduces a negative signal that has immunosuppressive effects. It further binds to several angiopoietin-like proteins (ANGPTLs), such as ANGPTL2 that promotes adaptive inflammation in tissue. A wide range of tumors express HLA-G, a nonclassical MHC-I molecule, which has been shown to mediate immunotolerance in cancer through the interaction with LILRB2, and thus allows such tumor cells to escape innate and adaptive immune response. Blocking of LILRB2 would hinder this escape mechanism. Besides, the blocking of LILRB2 can shift suppressed macrophages (M2) to activated state (M1-like).

There are several anti-LILRB2 antibodies known in the art. However, the number of LILRB2-BDs in the form of antibody fragments, such as scFvs, that can be used as universal building blocks for the construction of multispecific antibodies is limited. It is thus desirable to have suitable anti-LILRB2 antibody building blocks at hand that can readily be incorporated into any desirable multispecific antibody format, and that are capable of potently blocking the biological function of LILRB2. Furthermore, said building blocks should have superior biophysical properties, such as in particular a high stability, in order to facilitate their efficient incorporation into multispecific antibodies.

### SUMMARY OF THE INVENTION

It is an object of the present invention to provide novel and improved anti-LILRB2 antibody building blocks (LILRB2-BDs) that can readily be incorporated into any desirable multispecific antibody format, and that are capable of potently blocking the biological function of LILRB2. It is a further object of the present invention that said novel and improved LILRB2-BDs have superior biophysical properties, such as in particular a high stability, in order to facilitate their efficient incorporation into multispecific antibody therapeutics to foster developability and producibility in high yields.

The inventors have now surprisingly found that the LILRB2-BDs as defined herein can potently inhibit the biological function of LILRB2. In particular said LILRB2-BDs can potently block the interaction of LILRB2 to its ligands HLA-A3 and HLA-G. It has further been found that said LILRB2-BDs are capable to potently activate T-cells and stimulate re-polarization of M2 macrophages to their M1 state.

Said LILRB2-BDs also exhibit advantageous biophysical properties, in particular an outstanding stability, even when being present in the form of an antibody fragment, which allows high yield production and their successful incorporation in multispecific antibodies of different formats (see for example multispecific antibodies PRO4672, PRO5052, PRO4687 and PRO5055).

Accordingly, in a first aspect, the present invention relates to an antibody binding domain, which specifically binds to LILRB2 (LILRB2-BD), comprising:
a) a variable heavy chain (VH),
   wherein the variable heavy chain comprises, from N-terminus to C-terminus, the regions HFR1-HCDR1-HFR2-HCDR2-HFR3-HCDR3-HFR4, wherein each HFR designates a heavy chain framework region, and each HCDR designates a heavy chain complementarity-determining region, and
b) a variable light chain (VL),
wherein the variable light chain comprises, from N-terminus to C-terminus, the regions LFR1-LCDR1-LFR2-LCDR2-LFR3-LCDR3-LFR4, wherein each LFR designates a light chain framework region, and each LCDR designates a light chain complementarity-determining region,
wherein
said HCDR1 has the sequence of SEQ ID NO: 1,
said HCDR2 has the sequence of SEQ ID NO: 2,
said HCDR3 has the sequence of SEQ ID NO: 3,
said LCDR1 has the sequence of SEQ ID NO: 4,
said LCDR2 has the sequence of SEQ ID NO: 6, and
said LCDR3 has the sequence of SEQ ID NO: 7;
or wherein
said HCDR1 has the sequence of SEQ ID NO: 1,
said HCDR2 has the sequence of SEQ ID NO: 2,
said HCDR3 has the sequence of SEQ ID NO: 3,
said LCDR1 has the sequence of SEQ ID NO: 4,
said LCDR2 has the sequence of SEQ ID NO: 5, and
said LCDR3 has the sequence of SEQ ID NO: 7;
or wherein
said HCDR1 has the sequence of SEQ ID NO: 20,
said HCDR2 has the sequence of SEQ ID NO: 21,
said HCDR3 has the sequence of SEQ ID NO: 22,
said LCDR1 has the sequence of SEQ ID NO: 23,
said LCDR2 has the sequence of SEQ ID NO: 24, and
said LCDR3 has the sequence of SEQ ID NO: 26;
or wherein
said HCDR1 has the sequence of SEQ ID NO: 20,
said HCDR2 has the sequence of SEQ ID NO: 21,
said HCDR3 has the sequence of SEQ ID NO: 22,
said LCDR1 has the sequence of SEQ ID NO: 23,
said LCDR2 has the sequence of SEQ ID NO: 25, and
said LCDR3 has the sequence of SEQ ID NO: 26;
or wherein
said HCDR1 has the sequence of SEQ ID NO: 36,
said HCDR2 has the sequence of SEQ ID NO: 37,
said HCDR3 has the sequence of SEQ ID NO: 38,
said LCDR1 has the sequence of SEQ ID NO: 39,
said LCDR2 has the sequence of SEQ ID NO: 40, and
said LCDR3 has the sequence of SEQ ID NO: 42;
or wherein
said HCDR1 has the sequence of SEQ ID NO: 36,
said HCDR2 has the sequence of SEQ ID NO: 37,
said HCDR3 has the sequence of SEQ ID NO: 38,
said LCDR1 has the sequence of SEQ ID NO: 39,
said LCDR2 has the sequence of SEQ ID NO: 41, and
said LCDR3 has the sequence of SEQ ID NO: 42.

In a second aspect, the present invention relates to an antibody comprising two antibody binding domains of the present invention.

In a third aspect, the present invention relates to a multispecific antibody comprising:
a) one or two antibody variable domains of the present invention;
b) at least one binding domain, which specifically binds to a target different from LILRB2.

In a fourth aspect, the present invention relates to a nucleic acid or two nucleic acids encoding the antibody binding domain or the antibody of the present invention; or to a nucleic acid or two nucleic acids or three nucleic acids encoding the multispecific antibody of the present invention.

In a fifth aspect, the present invention relates to a vector or two vectors or three vectors comprising the nucleic acid or two nucleic acids or three nucleic acids of the present invention.

In a sixth aspect, the present invention relates to a host cell or host cells comprising the vector or the two vectors or the three vectors of the present invention.

In a seventh aspect, the present invention relates to a method for producing the antibody binding domain, or the antibody, or the multispecific antibody of the present invention, comprising (i) providing the nucleic acid or two nucleic acids or three nucleic acids of the present invention, or the vector or the two vectors or the three vectors of the present invention, expressing said nucleic acid or nucleic acids, or said vector or vectors, and collecting said multispecific antibody from the expression system, or (ii) providing a host cell or host cells of the present invention, culturing said host cell or said host cells; and collecting said multispecific antibody from the cell culture.

In an eighth aspect, the present invention relates to a pharmaceutical composition comprising the antibody of the present invention, or the multispecific antibody of the present invention, and a pharmaceutically acceptable carrier.

In a ninth aspect, the present invention relates to the antibody or the multispecific antibody of the present invention, or the pharmaceutical composition of the present invention for use as a medicament.

In a tenth aspect, the present invention relates to the antibody or the multispecific antibody of the present invention or the pharmaceutical composition of the present invention for use in the treatment of a disease, particularly a human disease, more particularly a human disease selected from cancer.

In an eleventh aspect, the present invention relates to a method for the treatment of a disease, particularly a human disease, more particularly a human disease selected from cancer, comprising the step of administering the antibody or the multispecific antibody of the present invention or the pharmaceutical composition of the present invention to a patient in need thereof.

The aspects, advantageous features and preferred embodiments of the present invention summarized in the following items, respectively alone or in combination, further contribute to solving the object of the invention:
1. An antibody binding domain, which specifically binds to LILRB2 (LILRB2-BD), comprising:
   a) a variable heavy chain (VH),
      wherein the variable heavy chain comprises, from N-terminus to C-terminus, the regions HFR1-HCDR1-HFR2-HCDR2-HFR3-HCDR3-HFR4, wherein each HFR designates a heavy chain framework region, and each HCDR designates a heavy chain complementarity-determining region, and
   b) a variable light chain (VL),
   wherein the variable light chain comprises, from N-terminus to C-terminus, the regions LFR1-LCDR1-LFR2-LCDR2-LFR3-LCDR3-LFR4, wherein each LFR designates a light chain framework region, and each LCDR designates a light chain complementarity-determining region,
   wherein
   said HCDR1 has the sequence of SEQ ID NO: 1,
   said HCDR2 has the sequence of SEQ ID NO: 2,
   said HCDR3 has the sequence of SEQ ID NO: 3,
   said LCDR1 has the sequence of SEQ ID NO: 4,
   said LCDR2 has the sequence of SEQ ID NO: 6, and
   said LCDR3 has the sequence of SEQ ID NO: 7;
   or wherein
   said HCDR1 has the sequence of SEQ ID NO: 1,
   said HCDR2 has the sequence of SEQ ID NO: 2,
   said HCDR3 has the sequence of SEQ ID NO: 3,
   said LCDR1 has the sequence of SEQ ID NO: 4,
   said LCDR2 has the sequence of SEQ ID NO: 5, and
   said LCDR3 has the sequence of SEQ ID NO: 7;
   or wherein
   said HCDR1 has the sequence of SEQ ID NO: 20,
   said HCDR2 has the sequence of SEQ ID NO: 21,
   said HCDR3 has the sequence of SEQ ID NO: 22,
   said LCDR1 has the sequence of SEQ ID NO: 23,
   said LCDR2 has the sequence of SEQ ID NO: 24, and
   said LCDR3 has the sequence of SEQ ID NO: 26;
   or wherein
   said HCDR1 has the sequence of SEQ ID NO: 20,
   said HCDR2 has the sequence of SEQ ID NO: 21,
   said HCDR3 has the sequence of SEQ ID NO: 22,
   said LCDR1 has the sequence of SEQ ID NO: 23,
   said LCDR2 has the sequence of SEQ ID NO: 25, and
   said LCDR3 has the sequence of SEQ ID NO: 26;
   or wherein
   said HCDR1 has the sequence of SEQ ID NO: 36,
   said HCDR2 has the sequence of SEQ ID NO: 37,
   said HCDR3 has the sequence of SEQ ID NO: 38,
   said LCDR1 has the sequence of SEQ ID NO: 39,
   said LCDR2 has the sequence of SEQ ID NO: 40, and
   said LCDR3 has the sequence of SEQ ID NO: 42;
   or wherein
   said HCDR1 has the sequence of SEQ ID NO: 36,
   said HCDR2 has the sequence of SEQ ID NO: 37,
   said HCDR3 has the sequence of SEQ ID NO: 38,
   said LCDR1 has the sequence of SEQ ID NO: 39,
   said LCDR2 has the sequence of SEQ ID NO: 41, and
   said LCDR3 has the sequence of SEQ ID NO: 42.
2. The antibody binding domain of item 1, wherein
   said HCDR1 has the sequence of SEQ ID NO: 1,
   said HCDR2 has the sequences of SEQ ID NO: 2,
   said HCDR3 has the sequence of SEQ ID NO: 3,
   said LCDR1 has the sequence of SEQ ID NO: 4,
   said LCDR2 has the sequence of SEQ ID NO: 6, and
   said LCDR3 has the sequences of SEQ ID NO: 7;
   or wherein
   said HCDR1 has the sequence of SEQ ID NO: 20,
   said HCDR2 has the sequences of SEQ ID NO: 21,
   said HCDR3 has the sequence of SEQ ID NO: 22,
   said LCDR1 has the sequence of SEQ ID NO: 23,
   said LCDR2 has the sequence of SEQ ID NO: 25, and
   said LCDR3 has the sequences of SEQ ID NO: 26;
   or wherein
   said HCDR1 has the sequence of SEQ ID NO: 36,
   said HCDR2 has the sequences of SEQ ID NO: 37,
   said HCDR3 has the sequence of SEQ ID NO: 38,
   said LCDR1 has the sequence of SEQ ID NO: 39,
   said LCDR2 has the sequence of SEQ ID NO: 41, and
   said LCDR3 has the sequences of SEQ ID NO: 42.
3. The antibody binding domain of item 1, wherein
   said HCDR1 has the sequence of SEQ ID NO: 1,
   said HCDR2 has the sequence of SEQ ID NO: 2,
   said HCDR3 has the sequence of SEQ ID NO: 3,
   said LCDR1 has the sequence of SEQ ID NO: 4,
   said LCDR2 has the sequence of SEQ ID NO: 5, and
   said LCDR3 has the sequence of SEQ ID NO: 7;
   or wherein
   said HCDR1 has the sequence of SEQ ID NO: 1,
   said HCDR2 has the sequence of SEQ ID NO: 2,
   said HCDR3 has the sequence of SEQ ID NO: 3,
   said LCDR1 has the sequence of SEQ ID NO: 4,
   said LCDR2 has the sequence of SEQ ID NO: 6, and
   said LCDR3 has the sequence of SEQ ID NO: 7.
4. The antibody binding domain of item 1, wherein
   said HCDR1 has the sequence of SEQ ID NO: 1,
   said HCDR2 has the sequence of SEQ ID NO: 2,
   said HCDR3 has the sequence of SEQ ID NO: 3,
   said LCDR1 has the sequence of SEQ ID NO: 4,
   said LCDR2 has the sequence of SEQ ID NO: 6, and
   said LCDR3 has the sequence of SEQ ID NO: 7.
5. The antibody binding domain of any one of the items 1 to 4, wherein said LILRB2-BD:
   a. when in scFv format, binds to human LILRB2 with a monovalent dissociation constant (KD) of 10 pM to 5 nM, particularly with a KD of 20 pM to 3 nM, particularly of 50 pM to 1 nM, as measured by surface plasmon resonance (SPR);
   b. blocks the interaction of LILRB2 to its ligand HLA-G with an IC₅₀ of 100 pM to 10 nM, particularly of 100 pM to 5 nM, particularly of 100 pM to 2 nM, as measured by flow cytometry;
   c. blocks the interaction of LILRB2 to its ligand HLA-A3 with an IC₅₀ of 100 pM to 10 nM, particularly of 100 pM to 5 nM, particularly of 100 pM to 2 nM, as measured by flow cytometry;
   d. is not cross-reactive with members of leukocyte immunoglobulin-like receptor subfamily A (LILRA); and/or
   e. is not cross reactive with members of leukocyte immunoglobulin-like receptor subfamily LILRB3, LILRB4 and LILRB5.
6. The antibody binding domain of any one of the items 1 to 5, wherein said LILRB2-BD:
   a. when in scFv format, has a melting temperature (Tm), determined by differential scanning fluorimetry, in the range of 55°C to 85°C, particularly in the range of 60°C to 85°C, in particular wherein said antibodies are formulated in 50 mM phosphate-citrate buffer at pH 6.4, 150 mM NaCl;
   b. when in scFv format, has a loss in monomer content, after storage for four weeks, at -80°C, of less than 5 %, less than 4 %, less than 3 %, less than 2 %, particularly less than 1.5 %, when said antibodies are at a starting concentration of 10 mg/ml, and in particular wherein said antibody is formulated in 50 mM phosphate citrate buffer with 150 mM NaCl at pH 6.4;
   c. when in scFv format, has a loss in monomer content, after storage for four weeks, at 4°C, of less than 15 %, e.g. less than 12 %, less than 10 %, less than 9 %, less than 8 %, particularly less than 7 %, when said antibodies are at a starting concentration of 10 mg/ml, and in particular wherein said antibodies are formulated in 50 mM phosphate citrate buffer with 150 mM NaCl at pH 6.4;
   d. when in scFv format, has a loss in monomer content, after storage for four weeks, at 40°C, of less than 15 %, e.g. less than 12 %, less than 10 %, less than 9 %, less than 8 %, particularly less than 7 %, when said antibodies are at a starting concentration of 10 mg/ml, and in particular wherein said antibodies are formulated in 50 mM phosphate citrate buffer with 150 mM NaCl at pH 6.4.
7. The antibody binding domain of any one of the items 1 to 6, wherein said LILRB2-BD is in a format selected from a Fab, an scFab, an Fv, a dsFv, an scFv and a dsscFv, particularly from a Fab, Fv and scFv.
8. The antibody binding domain of any one of the items 1 to 6, wherein said LILRB2-BD is in a format selected from an Fv, a dsFv, an scFv and a dsscFv, particularly from an Fv and scFv.
9. The antibody binding domain of any one of the items 1 to 8, wherein said HFR1, HFR2, HFR3 and HFR4 are selected from VH3 or VH4 domain framework sequences, particularly from VH3 domain framework sequences.
10. The antibody binding domain of any one of the items 1 to 9, wherein
   a) said LFR1, LFR2, LFR3 and LFR4 are selected from Vκ subtypes, particularly from the Vκ1 and Vκ3 subtypes, particularly are of the Vκ1 subtype; or
   b) said LFR1, LFR2 and LFR3 are selected from Vκ subtypes, particularly from the Vκ1 and Vκ3 subtypes, particularly are of the Vκ1 subtype, and said LFR4 is selected from a Vλ framework 4, particularly is a Vλ framework 4 comprising an amino acid sequence having at least 80, 90 percent identity to any of SEQ ID NOs: 62 to 70, more particularly a Vλ framework 4 selected from any of SEQ ID NOs: 62 to 70, particularly has a Vλ framework 4 according to SEQ ID NO: 62 or 70.
11. The antibody binding domain of any one of the items 1 to 10, comprising:
   a) a VH sequence at least 90, 91, 92, 93, 94, 95, 96, 97, 98 or 99 percent identical to the amino acid sequence of SEQ ID NO: 8; and
      a VL sequence at least 90, 91, 92, 93, 94, 95, 96, 97, 98 or 99 percent identical to any one of the amino acid sequences selected from SEQ ID NOs: 12, 14 and 15, particularly selected from SEQ ID NOs: 12 and 15, particularly to SEQ ID NO: 15; or
   b) a VH sequence at least 90, 91, 92, 93, 94, 95, 96, 97, 98 or 99 percent identical to the amino acid sequence of SEQ ID NO: 9; and
      a VL sequence at least 90, 91, 92, 93, 94, 95, 96, 97, 98 or 99 percent identical to any one of the amino acid sequences selected from SEQ ID NOs: 11 and 12, particularly to SEQ ID NO: 12; or
   c) a VH sequence at least 90, 91, 92, 93, 94, 95, 96, 97, 98 or 99 percent identical to the amino acid sequence of SEQ ID NO: 10; and
      a VL sequence at least 90, 91, 92, 93, 94, 95, 96, 97, 98 or 99 percent identical to the amino acid sequence of SEQ ID NO: 13; or
   d) a VH sequence at least 90, 91, 92, 93, 94, 95, 96, 97, 98 or 99 percent identical to the amino acid sequence of SEQ ID NO: 27; and
      a VL sequence at least 90, 91, 92, 93, 94, 95, 96, 97, 98 or 99 percent identical to any one of the amino acid sequences selected from SEQ ID NOs: 30, 31 and 32, particularly selected from SEQ ID NOs: 30 and 32, particularly to SEQ ID NO: 32; or
   e) a VH sequence at least 90, 91, 92, 93, 94, 95, 96, 97, 98 or 99 percent identical to the amino acid sequence of SEQ ID NO: 28; and
      a VL sequence at least 90, 91, 92, 93, 94, 95, 96, 97, 98 or 99 percent identical to any one of the amino acid sequences selected from SEQ ID NOs: 29 and 30, particularly to SEQ ID NO: 30; or
   f) a VH sequence at least 90, 91, 92, 93, 94, 95, 96, 97, 98 or 99 percent identical to the amino acid sequence of SEQ ID NO: 43; and
      a VL sequence at least 90, 91, 92, 93, 94, 95, 96, 97, 98 or 99 percent identical to any one of the amino acid sequences selected from SEQ ID NOs: 46, 47 and 48, particularly selected from SEQ ID NOs: 46 and 48, particularly to SEQ ID NO: 48; or
   g) a VH sequence at least 90, 91, 92, 93, 94, 95, 96, 97, 98 or 99 percent identical to the amino acid sequence of SEQ ID NO: 44; and
      a VL sequence at least 90, 91, 92, 93, 94, 95, 96, 97, 98 or 99 percent identical to the amino acid sequence of SEQ ID NO: 46.
12. The antibody binding domain of any one of the items 1 to 10, comprising:
   a) a VH sequence of SEQ ID NO: 8; and
      a VL sequence selected from SEQ ID NOs: 12, 14 and 15, particularly selected from SEQ ID NOs: 12 and 15, particularly from SEQ ID NO: 15; or
   b) a VH sequence of SEQ ID NO: 9; and
      a VL sequence selected from SEQ ID NOs: 11 and 12, particularly from SEQ ID NO: 12; or
   c) a VH sequence of SEQ ID NO: 10; and
      a VL sequence of SEQ ID NO: 13; or
   d) a VH sequence of SEQ ID NO: 27; and
      a VL sequence selected from SEQ ID NOs: 30, 31 and 32, particularly selected from SEQ ID NOs: 30 and 32, particularly from SEQ ID NO: 32; or
   e) a VH sequence of SEQ ID NO: 28; and
      a VL sequence selected from SEQ ID NOs: 29 and 30, particularly from SEQ ID NO: 30; or
   f) a VH sequence of SEQ ID NO: 43; and
      a VL sequence selected from SEQ ID NOs: 46, 47 and 48, particularly from SEQ ID NOs: 46 and 48, particularly from SEQ ID NO: 48; or
   g) a VH sequence of SEQ ID NO: 44; and
      a VL sequence of SEQ ID NO: 46.
13. The antibody binding domain of any one of the preceding items, which is an scFv antibody fragments having a sequence selected from SEQ ID NOs: 16, 17, 18, 19, 33, 34, 35, 49, 50 and 51.
14. An monospecific antibody comprising two antibody binding domains according to any one of the items 1 to 12.
15. The antibody of item 14, which has amino acid sequences selected from:
   - SEQ ID NO: 75 and SEQ ID NO: 76 (PRO3603);
   - SEQ ID NO: 77 and SEQ ID NO: 78 (PRO3635); and
   - SEQ ID NO: 79 and SEQ ID NO: 80 (PRO4710).
16. A multispecific antibody comprising:
   a) one or two antibody variable domains as defined in any one of items 1 to 13;
   b) at least one binding domain, which specifically binds to a target different from LILRB2.
17. The multispecific antibody of item 16, wherein the multispecific antibody does not comprise an immunoglobulin Fc region.
18. The multispecific antibody of item 17, wherein the multispecific antibody is in a format selected from the group consisting of: a tandem scDb (Tandab), a linear dimeric scDb (LD-scDb), a circular dimeric scDb (CD-scDb), a tandem tri-scFv, a tribody (Fab-(scFv)2), a Fab-Fv2, a triabody, an scDb-scFv, a tetrabody, a didiabody, a tandem-di-scFv and a MATCH.
19. The multispecific antibody of item 16, wherein the multispecific antibody comprises an immunoglobulin Fc region.
20. The multispecific antibody of item 19, wherein the immunoglobulin Fc region is an active Fc region.
21. The multispecific antibody of item 19 or 20, wherein the immunoglobulin Fc region has one or more amino acid modifications selected from the group consisting of:
   a) modifications to enhance heterodimerization of heavy chains, such as the knob-into-hole (KiH) technology;
   b) modifications that increase or decrease Fc receptor binding, when compared to the respective unmodified immunoglobulin Fc region thereof;
   c) modifications that increase serum half-life, when compared to the respective unmodified immunoglobulin Fc region thereof.
22. The multispecific antibody of item 19, wherein the immunoglobulin Fc region is selected from an IgG subclass, particularly from IgG subclasses IgG1.
23. The multispecific antibody of any one of the items 16 to 22, which has amino acid sequences selected from:
   - SEQ ID NO: 81 and SEQ ID NO: 82 (PRO4672);
   - SEQ ID NO: 83, SEQ ID NO: 84 and SEQ ID NO: 85 (PRO4687);
   - SEQ ID NO: 86 and SEQ ID NO: 87 (PRO5052); and
   - SEQ ID NO: 88, SEQ ID NO: 89 and SEQ ID NO: 90 (PRO5055).
24. A nucleic acid or two nucleic acids encoding the antibody binding domain of any one of items 1 to 13, or the antibody of any one of the items 14 or 15; or a nucleic acid or two nucleic acids or three nucleic acids encoding the multispecific antibody of any one of the items 16 to 23.
25. A vector or two vectors or three vectors comprising the nucleic acid or two nucleic acids or three nucleic acids of item 24.
26. A host cell or host cells comprising the vector or the two vectors or the three vectors of item 25.
27. A method for producing the antibody binding domain of any one of items 1 to 13, or the antibody of any one of the items 14 or 15, or the multispecific antibody of any one of the items 16 or 23, comprising (i) providing the nucleic acid or two nucleic acids or three nucleic acids of item 24, or the vector or the two vectors or the three vectors of item 25, expressing said nucleic acid or nucleic acids, or said vector or vectors, and collecting said multispecific antibody from the expression system, or (ii) providing a host cell or host cells according to item 25, culturing said host cell or said host cells; and collecting said multispecific antibody from the cell culture.
28. A pharmaceutical composition comprising the antibody of item 14 or 15, or the multispecific antibody of any one of the items 16 or 23, and a pharmaceutically acceptable carrier.
29. The antibody of item 14 or 15, or the multispecific antibody of any one of items 16 to 23, or the pharmaceutical composition of item 28, for use as a medicament.
30. The antibody of item 14 or 15 or the multispecific antibody of any one of items 16 to 23 or the pharmaceutical composition of item 28 for use in the treatment of a disease, particularly a human disease, more particularly a human disease selected from cancer.
31. The antibody or multispecific antibody or pharmaceutical composition of item 30,
   wherein the cancer is selected from
   a benign or especially malignant tumor, solid tumors, mesothelioma, brain cancer, kidney cancer, liver cancer, adrenal gland cancer, bladder cancer, breast cancer, stomach cancer (e. g., gastric tumors), esophageal cancer, ovarian cancer, cervical cancer, thymic cancer, choriocarcinoma, oral cancer, salivary cancer, colon cancer, rectum cancer, prostate cancer, pancreatic cancer, lung cancer (e. g., non-small cell lung cancer and small cell lung cancer), vaginal cancer, thyroid cancer, melanoma (e. g., unresectable or metastatic melanoma), renal cell carcinoma, sarcoma, glioma, glioblastoma, multiple myeloma or gastrointestinal cancer, especially colon carcinoma or colorectal adenoma, a tumor of the neck and head, endometrial cancer, Cowden syndrome, Lhermitte-Duclos disease, Bannayan-Zonana syndrome, prostate hyperplasia, a neoplasia, especially of epithelial character, preferably mammary carcinoma or squamous cell carcinoma, chronic lymphocytic leukemia, chronic myelogenous leukemia (e. g., Philadelphia chromosome-positive chronic myelogenous leukemia), acute lymphoblastic leukemia (e. g., Philadelphia chromosome-positive acute lymphoblastic leukemia), non-Hodgkin's lymphoma, plasma cell myeloma, Hodgkin's lymphoma, a leukemia, and any combination thereof.
32. A method for the treatment of a disease, particularly a human disease, more particularly a human disease selected from cancer, comprising the step of administering the antibody of item 14 or 15 or the multispecific antibody of any one of items 16 to 23 or the pharmaceutical composition of item 28 to a patient in need thereof.
33. The method of item 32, wherein the cancer is selected from
   a benign or especially malignant tumor, solid tumors, mesothelioma, brain cancer, kidney cancer, liver cancer, adrenal gland cancer, bladder cancer, breast cancer, stomach cancer (e. g., gastric tumors), esophageal cancer, ovarian cancer, cervical cancer, thymic cancer, choriocarcinoma, oral cancer, salivary cancer, colon cancer, rectum cancer, prostate cancer, pancreatic cancer, lung cancer (e. g., non-small cell lung cancer and small cell lung cancer), vaginal cancer, thyroid cancer, melanoma (e. g., unresectable or metastatic melanoma), renal cell carcinoma, sarcoma, glioma, glioblastoma, multiple myeloma or gastrointestinal cancer, especially colon carcinoma or colorectal adenoma, a tumor of the neck and head, endometrial cancer, Cowden syndrome, Lhermitte-Duclos disease, Bannayan-Zonana syndrome, prostate hyperplasia, a neoplasia, especially of epithelial character, preferably mammary carcinoma or squamous cell carcinoma, chronic lymphocytic leukemia, chronic myelogenous leukemia (e. g., Philadelphia chromosome-positive chronic myelogenous leukemia), acute lymphoblastic leukemia (e. g., Philadelphia chromosome-positive acute lymphoblastic leukemia), non-Hodgkin's lymphoma, plasma cell myeloma, Hodgkin's lymphoma, a leukemia, and any combination thereof.

### BRIEF DESCRIPTION OF THE DRAWINGS

**Figure 1** shows the binding properties of PRO4710 (IgG4 of scFv PRO3744, 68-18-E11-sc07) to members of LILRBA and LILRB family expressed by CHO cells.
Figure 2 shows the binding properties of PRO3635 (IgG of scFv PRO3064, 68-18-E11-sc02) and the references PRO3483 (NGM), PRO2518 (J19) and PRO3848 (Biond) to members of LILRA family expressed by CHO cells. An anti-V5 tag antibody served as positive control as all constructs contain V5 tag at N-terminus.
Figure 3 shows the binding properties of PRO3635 (IgG of scFv PRO3064, 68-18-E11-sc02) and the references PRO3483 (NGM), PRO2518 (J19) and PRO3848 (Biond) to members of LILRB family expressed by CHO cells. An anti-V5 tag antibody served as positive control as all constructs contain V5 tag at N-terminus.
Figure 4 shows the binding of the scFv PRO3153 (69-01-E02-sc03) applied at concentrations of 150 nM (A) and 50 nM (B) to members of LILRBA and LILRB family expressed by CHO cells.
Figure 5 shows the binding properties of the scFv PRO3697 (69-06-G04-sc05), cross-linked with an anti-framework antibody (x-linked), to members of LILRBA and LILRB family expressed by CHO cells.
Figure 6 shows the dose-dependent increase of the M1 marker CD86 and the dose-dependent decrease of the M2 marker CD163 in the presence of increasing concentrations of PRO3744, PRO3696 and PRO3697.
Figure 16 shows the T-cell proliferation potency of the anti-LILRB2 antibody PRO4710, the trispecific antibodies PRO5052, PRO5053 (no-LILRB2-BD), PRO5055, PRO5056 (no-LILRB2-BD), PRO5054 (no CD47-BD), and the reference antibodies avelumab and magrolimab.
Figure 23 shows the binding properties of PRO5052 (A) and PRO5055 (B) to members of LILRBA and LILRB family expressed by CHO cells.

### DETAILED DESCRIPTION OF THE INVENTION

The number of available LILRB2-BDs in the form of antibody fragments, such as scFvs, that can be used as universal building blocks for the construction of multispecific antibodies is limited. There is thus a need in the medical field to provide novel and improved anti-LILRB2 antibody building blocks (LILRB2-BDs) that can readily be incorporated into any desirable multispecific antibody format, and that are capable of potently blocking the biological function of LILRB2.

The present invention provides LILRB2-BDs that can potently inhibit the biological function of LILRB2. In particular said LILRB2-BDs can potently block the interaction of LILRB2 to its ligands HLA-A3 and HLA-G. The LILRB2-BDs of the present invention thus have the potency to hinder cancer cells, which express these ligands, to escape innate and adaptive immune response. Furthermore, said LILRB2-BDs are capable of potently activating T-cells and stimulating re-polarization of M2 macrophages to their M1 state.

The LILRB2-BDs of the present invention also exhibit advantageous biophysical properties, in particular an outstanding stability, even when being present in the form of an antibody fragment, for example an scFv, which allows high yield production and their successful incorporation in multispecific antibodies of different formats. For example, the LIRB2-BD of the present invention could be successfully incorporated as scFvs into the multispecific molecules PRO4672, PRO5052, PRO4687 and PRO5055.

Unless defined otherwise, all technical and scientific terms used herein have the same meaning as commonly understood by those of ordinary skill in the art to which this invention pertains.

The terms "comprising" and "including" are used herein in their open-ended and non-limiting sense unless otherwise noted. With respect to such latter embodiments, the term "comprising" thus includes the narrower term "consisting of".

The terms "a" and "an" and "the" and similar references in the context of describing the invention (especially in the context of the following claims) are to be construed to cover both the singular and the plural, unless otherwise indicated herein or clearly contradicted by context. For example, the term "a cell" includes a plurality of cells, including mixtures thereof. Where the plural form is used for compounds, salts, and the like, this is taken to mean also a single compound, salt, or the like.

The term "antibody" and the like, as used herein, includes whole antibodies or single chains thereof; and any antigen-binding fragment (i. e., "antigen-binding portion") or single chains thereof; and molecules comprising antibody CDRs, VH regions or VL regions (including without limitation multispecific antibodies). A naturally occurring "whole antibody" is a glycoprotein comprising at least two heavy (H) chains and two light (L) chains interconnected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDRs), flanked by regions that are more conserved, termed framework regions (FRs). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e. g., effector cells) and the first component (C1q) of the classical complement system.

The term "immunoglobulin Fc region" or "Fc region", as used herein, is used to define a C-terminal region of an immunoglobulin heavy chain, i. e. the CH2 and CH3 domains of the heavy chain constant regions. The Fc region provides linkage to humoral and cellular components of the immune system, mostly through interaction with C1q and Fc receptors (FcR). The term "Fc region" includes Fc regions of an IgG subclass, particularly an immunoglobulin Fc region of human IgG1, IgG2 (IgG2A, IgG2B), IgG3 or IgG4. Further included are Fc regions that are engineered, i. e. Fc regions that have one or more amino acid modifications, to exhibit certain desired properties. Such amino acid modifications are for example modifications to enhance heterodimerization of heavy chains, such as knob-into-hole (KiH) technology (see for example Ridgway et al., Protein Eng. 9:617-21 (1996) and Spiess et al., J Biol Chem. 288(37):26583-93 (2013)). Other amino acid modifications are modifications that alter, i.e. increase or decrease Fc receptor binding, when compared to the respective unmodified immunoglobulin Fc region thereof (see for example the modifications disclosed in WO 2004/42072 (Presta) and in Wang et al., Protein Cell. 9(1):63-732018 (2018)) and/or increase serum half-life (see for example the modifications disclosed in Liu et al., Antibodies (Basel). 9(4):64 (2020)).

The term "Fc receptor" or "FcR" describes a receptor that binds to the Fc region of an antibody. Particularly, the Fc receptors are native sequence human FcRs, which binds an IgG antibody. These include for example the Fc gamma receptors (FcγRs). Five activating FcyRs are known, i. e. the high affinity receptors FcγRl, and the lower affinity receptors FcγRIIA, FcγRIIC, FcγRIIIA and FcγRIIIB. Besides, one inhibiting receptor is known, i. e. FcγRIIB, which have similar amino acid sequences to FcγRIIA that differ primarily in the cytoplasmic domains thereof. Activating receptors contain an immunoreceptor tyrosine-based activation motif (ITAM) in its cytoplasmic domain. The inhibiting receptor FcγRIIB contains an immunoreceptor tyrosine-based inhibition motif (ITIM) in its cytoplasmic domain, (see M. Daeron, Annu. Rev. Immunol. 5:203-234 (1997). FcRs are reviewed for example in Ravetch and Kinet, Annu. Rev. Immunol. 9: 457-92 (1991); Capet et al, Immunomethods 4: 25-34 (1994); and de Haas et al, J. Lab. Clin. Med. 126: 330-41 (1995). Other FcRs, including those to be identified in the future, are encompassed by the term "FcR" herein. The term "Fc receptor" or "FcR" also includes the neonatal receptor FcRn, which plays a central role in the cellular trafficking and serum half-life of IgGs. High-affinity binding to FcRn *in vivo* is reported to prolong serum half-life of human IgGs (see for example Dall'Acqua et al., J Biol Chem. 281:23514-23524(2006)).

The term "silenced Fc", "Fc silenced" or "silenced Fc region", as used herein, refers to an Fc region, which has been engineered to exhibit insignificant or negligible Fc receptor-binding functionality, in particular insignificant or negligible binding affinity to FcyRs, when compared to its unmodified version. Antibodies having such silenced Fc regions do not or do not significantly trigger cellular immune responses through Fc receptor binding.

The term "active Fc" or "active Fc region", as used herein, refers to an Fc region, which has significant Fc receptor binding functionality, in particular significant binding affinity to FcyRs. This Fc receptor binding functionality may be present naturally and/or may be introduced or enhanced by engineering. Antibodies having such active Fc regions trigger cellular immune responses through Fc receptor binding.

The terms "binding domain", "antigen-binding fragment thereof", "antigen-binding portion" of an antibody, and the like, as used herein, refer to one or more fragments of an intact antibody that retain the ability to specifically bind to a given antigen (e. g., LILRB2). Antigen-binding functions of an antibody can be performed by fragments of an intact antibody.

Specifically, in case of the antibody binding domain of the present invention, the terms "binding domain", "antigen-binding fragment", "antigen-binding fragment thereof", "antigen-binding portion", and the like, as used herein, refer to a Fab fragment, i. e. a monovalent fragment consisting of the VL, VH, CL and CH1 domains; a single chain Fab (scFab), i. e. a Fab fragment, where the heavy chain and the light chain are connected via a linker; an Fv fragment consisting of the VL and VH domains of a single arm of an antibody; a disulfide stabilized Fv fragment (dsFv); and a single chain Fv fragment (scFv). The scFv can also be stabilized by a disulfide bridge (dsscFv).

In particular embodiments, the antibody binding domains, i. e. LILRB2-BDs, of the present invention are selected from a Fab, an scFab, an Fv, a dsFv, an scFv and a dsscFv, particularly from a Fab, an Fv and an scFv.

In other particular embodiments, the antibody binding domains, i. e. LILRB2-BDs, of the present invention are selected from an Fv, a dsFv, an scFv and a dsscFv, particularly from an Fv and an scFv.

In other particular embodiments, the VL and VH domains of the scFv fragment are stabilized by an interdomain disulfide bond (dsscFv), in particular said VH domain comprises a single cysteine residue in position 51 (AHo numbering) and said VL domain comprises a single cysteine residue in position 141 (AHo numbering).

The term "Complementarity Determining Regions" ("CDRs") refers to amino acid sequences with boundaries determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273, 927-948 ("Chothia" numbering scheme); ImMunoGenTics (IMGT) numbering (Lefranc, M.-P., The Immunologist, 7, 132-136 (1999); Lefranc, M.-P. et al., Dev. Comp. Immunol., 27, 55-77 (2003)) ("IMGT" numbering scheme); and the numbering scheme described in Honegger & Plückthun, J. Mol. Biol. 309 (2001) 657-670 ("AHo" numbering). For example, for classic formats, under Kabat, the CDR amino acid residues in the heavy chain variable region (VH) are numbered 31-35 (HCDR1), 50-65 (HCDR2), and 95-102 (HCDR3); and the CDR amino acid residues in the light chain variable region (VL) are numbered 24-34 (LCDR1), 50-56 (LCDR2), and 89-97 (LCDR3). Under Chothia the CDR amino acids in the VH are numbered 26-32 (HCDR1), 52-56 (HCDR2), and 95-102 (HCDR3); and the amino acid residues in VL are numbered 24-34 (LCDR1), 50-56 (LCDR2), and 89-97 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. Under IMGT the CDR amino acid residues in the VH are numbered approximately 26-35 (HCDR1), 51-57 (HCDR2) and 93-102 (HCDR3), and the CDR amino acid residues in the VL are numbered approximately 27-32 (LCDR1), 50-52 (LCDR2), and 89-97 (LCDR3) (numbering according to "Kabat"). Under IMGT, the CDRs of an antibody can be determined using the program IMGT/DomainGap Align.

In the context of the present invention, the numbering system suggested by Honegger & Plückthun ("AHo") is used (Honegger & Plückthun, J. Mol. Biol. 309 (2001) 657-670), unless specifically mentioned otherwise. In particular, the following residues are defined as CDRs according to AHo numbering scheme: LCDR1 (also referred to as CDR-L1): L24-L42; LCDR2 (also referred to as CDR-L2): L58-L72; LCDR3 (also referred to as CDR-L3): L107-L138; HCDR1 (also referred to as CDR-H1): H27-H42; HCDR2 (also referred to as CDR-H2): H57-H76; HCDR3 (also referred to as CDR-H3): H108-H138. For the sake of clarity, the numbering system according to Honegger & Plückthun takes the length diversity into account that is found in naturally occurring antibodies, both in the different VH and VL subfamilies and, in particular, in the CDRs, and provides for gaps in the sequences. Thus, in a given antibody variable region usually not all positions 1 to 149 will be occupied by an amino acid residue.

The term "binding specificity" as used herein refers to the ability of an individual antibody to react with one antigenic determinant and not with a different antigenic determinant. As used herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In its most general form (and when no defined reference is mentioned), "specific binding" is referring to the ability of the antibody to discriminate between the target of interest and an unrelated molecule, as determined, for example, in accordance with specificity assay methods known in the art. Such methods comprise, but are not limited to Western blots, ELISA, RIA, ECL, IRMA, SPR (Surface plasmon resonance) tests and peptide scans. For example, a standard ELISA assay can be carried out. The scoring may be carried out by standard color development (e. g. secondary antibody with horseradish peroxide and tetramethyl benzidine with hydrogen peroxide). The reaction in certain wells is scored by the optical density, for example, at 450 nm. Typical background (= negative reaction) may be about 0.1 OD; typical positive reaction may be about 1 OD. This means the ratio between a positive and a negative score can be 10-fold or higher. In a further example, an SPR assay can be carried out, wherein at least 10-fold, particularly at least 100-fold difference between a background and signal indicates on specific binding. Typically, determination of binding specificity is performed by using not a single reference molecule, but a set of about three to five unrelated molecules, such as milk powder, transferrin or the like.

Suitably, the antibody binding domains and antibodies or multispecific antibodies of the invention are isolated antibodies. The term "isolated antibody", as used herein, refers to an antibody that is substantially free of other antibodies having different antigenic specificities (e. *g.,* an isolated antibody that specifically binds LILRB2 is substantially free of antibodies that specifically bind antigens other than LILRB2). Moreover, an isolated antibody may be substantially free of other cellular material and/or chemicals.

Suitably, the antibody binding domains and antibodies or multispecific antibodies of the invention are monoclonal antibodies. The term "monoclonal antibody" as used herein refers to antibodies that have substantially identical amino acid sequences or are derived from the same genetic source. A monoclonal antibody displays a binding specificity and affinity for a particular epitope, or binding specificities and affinities for specific epitopes.

The antibody binding domains and antibodies or multispecific antibodies of the invention include, but are not limited to, chimeric, human and humanized antibody binding domains and antibodies.

The term "chimeric antibody binding domain" or "chimeric antibody", as used herein, refers to an antibody binding domains or antibody or multispecific antibody molecule in which (a) the constant region, or a portion thereof, is altered, replaced or exchanged so that the antigen-binding site (variable region) is linked to a constant region of a different or altered class, effector function and/or species; or (b) the variable region, or a portion thereof, is altered, replaced or exchanged with a variable region having a different or altered antigen specificity. For example, a mouse antibody can be modified by replacing its constant region with the constant region from a human immunoglobulin. Due to the replacement with a human constant region, the chimeric antibody can retain its specificity in recognizing the antigen while having reduced antigenicity in human as compared to the original mouse antibody.

The term "human antibody binding domain" or "human antibody", as used herein, is intended to include antibody binding domains and antibodies or multispecific antibodies having variable regions in which both the framework and CDR regions are derived from sequences of human origin. Furthermore, if the antibody contains a constant region, the constant region also is derived from such human sequences, e. g., human germline sequences, or mutated versions of human germline sequences. The human antibody binding domains and antibodies or multispecific antibodies of the invention may include amino acid residues not encoded by human sequences (e. g., mutations introduced by random or site-specific mutagenesis *in vitro* or by somatic mutation *in vivo).* This definition of a human antibody binding domain and human antibody specifically excludes a humanized antibody comprising non-human antigen-binding residues. Human antibody binding domains and antibodies can be produced using various techniques known in the art, including phage-display libraries (Hoogenboom and Winter, J. Mol. Biol, 227:381 (1992); Marks et al, J. Mol. Biol, 222:581 (1991)). Also available for the preparation of human monoclonal antibody binding domains and antibodies are methods described in Cole et al, Monoclonal Antibodies and Cancer Therapy, Alan R. Liss, p. 77 (1985); Boemer et al, J. Immunol, 147(I):86-95 (1991). See also van Dijk and van de Winkel, Curr. Opin. Pharmacol, 5: 368-74 (2001). Human antibody binding domains and antibodies can be prepared by administering the antigen to a transgenic animal that has been modified to produce such antibodies in response to antigenic challenge, but whose endogenous loci have been disabled, e. *g.,* immunized xenomice (see, e. g., U.S. Pat. Nos. 6,075,181 and 6,150,584 regarding XENOMOUSE^{™} technology). See also, for example, Li et al, Proc. Natl. Acad. Sci. USA, 103:3557- 3562 (2006) regarding human antibodies generated via a human B-cell hybridoma technology.

The term "humanized" antibody binding domain or "humanized" antibodies as used herein, refers to an antibody binding domain or an antibody or multispecific antibody that retains the reactivity of a non-human antibody binding domain or antibody while being less immunogenic in humans. This can be achieved, for instance, by retaining the non-human CDR regions and replacing the remaining parts of the antibody binding domain or antibody with their human counterparts (i. e., the constant region as well as the framework portions of the variable region). Additional framework region modifications may be made within the human framework sequences as well as within the CDR sequences derived from the germline of another mammalian species. In particular, it is known that CDR1 of VH and CDR2 of VL are less important for binding specificity, as evidenced by the fact that in many antibodies obtained by immunization, those CDRs are identical to the CDR encoded by the corresponding germline gene segment. Thus, in one embodiment of the invention, the antibody or antigen-binding fragment thereof comprises in CDR1 of VH and/or in CDR2 of VL one or two amino acid residues that differ from the amino acid residues of the parental antibody. The humanized antibody binding domain and antibodies and multispecific antibodies of the invention may include amino acid residues not encoded by human sequences (e. *g.,* mutations introduced by random or site-specific mutagenesis *in vitro* or by somatic mutation *in vivo,* or a conservative substitution to promote stability or manufacturing). See, e. g., Morrison et al., Proc. Natl. Acad. Sci. USA, 81:6851-6855, 1984; Morrison and Oi, Adv. Immunol., 44:65-92, 1988; Verhoeyen et al., Science, 239: 1534-1536, 1988; Padlan, Molec. Immun., 28:489-498, 1991; and Padlan, Molec. Immun., 31: 169-217, 1994. Other examples of human engineering technology include but are not limited to the Xoma technology disclosed in U.S. Pat. No. 5,766,886.

Preferably, the antibody binding domains and antibodies and multispecific antibodies of the invention are humanized. More preferably, the antibody binding domains and antibodies and multispecific antibodies of the invention are humanized and comprises rabbit derived CDRs.

The term "monospecific antibody" as used herein, refers to an antibody that binds to one or more different epitopes on the same target (e. g. LILRB2).

The term "multispecific antibody" as used herein, refers to an antibody that binds to two or more different epitopes on at least two or more different targets (e. g., CD47, PDL1, hSA and LILRB2). Preferably, the multispecific antibodies of the invention are bispecific or trispecific. The term "bispecific antibody" as used herein, refers to an antibody that binds to at least two different epitopes on two different targets (e. g., LILRB2 and PDL1). The term "trispecific antibody" as used herein, refers to an antibody that binds to at least three different epitopes on three different targets (e. g., CD47, PDL1 and LILRB2).

The term "epitope" means a protein determinant capable of specific binding to an antibody binding domain or an antibody. Epitopes usually consist of chemically active surface groupings of molecules such as amino acids or sugar side chains and usually have specific three-dimensional structural characteristics, as well as specific charge characteristics. "Conformational" and "linear" epitopes are distinguished in that the binding to the former but not the latter is lost in the presence of denaturing solvents.

The term "conformational epitope" as used herein refers to amino acid residues of an antigen that come together on the surface when the polypeptide chain folds to form the native protein.

The term "linear epitope" refers to an epitope, wherein all points of interaction between the protein and the interacting molecule (such as an antibody) occurring linearly along the primary amino acid sequence of the protein (continuous).

The term "recognize" as used herein refers to an antibody antigen-binding fragment thereof that finds and interacts (e. g., binds) with its conformational epitope.

The term "avidity" as used herein refers to an informative measure of the overall stability or strength of the antibody-antigen complex. It is controlled by three major factors: antibody epitope affinity; the valency of both the antigen and antibody; and the structural arrangement of the interacting parts. Ultimately these factors define the specificity of the antibody, that is, the likelihood that the particular antibody is binding to a precise antigen epitope.

Suitably, the LIRB2-BD of the invention targets in particular human LILRB2. Particularly, the LIRB2-BD of the invention targets human and cynomolgus (Macaca *fascicularis)* LI LR B2.

Suitably, the LILRB2-BD of the present invention is characterized by one or more of the following parameters:
a. when in scFv format, binds to human LILRB2 with a monovalent dissociation constant (KD) of 10 pM to 5 nM, particularly with a KD of 20 pM to 3 nM, particularly of 50 pM to 1 nM, as measured by surface plasmon resonance (SPR);
b. blocks the interaction of LILRB2 to its ligand HLA-G with an IC₅₀ of 100 pM to 10 nM, particularly of 100 pM to 5 nM, particularly of 100 pM to 2 nM, as measured by flow cytometry;
c. blocks the interaction of LILRB2 to its ligand HLA-A3 with an IC₅₀ of 100 pM to 10 nM, particularly of 100 pM to 5 nM, particularly of 100 pM to 2 nM, as measured by flow cytometry;
d. is not cross-reactive with members of leukocyte immunoglobulin-like receptor subfamily A (LILRA); and/or
e. is not cross reactive with members of leukocyte immunoglobulin-like receptor subfamily LILRB3, LILRB4 and LILRB5.

As used herein, the term "affinity" refers to the strength of interaction between a molecule (e. g., of an antibody) and the antigen at single antigenic sites. Within each antigenic site, the variable region of the antibody "arm" interacts through weak non-covalent forces with antigen at numerous sites; the more interactions, the stronger the affinity.

"Binding affinity" generally refers to the strength of the total sum of non-covalent interactions between a single binding site of a molecule (e. g., of an antibody) and its binding partner (e. *g.,* an antigen or, more specifically, an epitope on an antigen). Unless indicated otherwise, as used herein, "binding affinity", "bind to", "binds to" or "binding to" refers to intrinsic binding affinity that reflects a 1:1 interaction between members of a binding pair (e. g., an antibody fragment and an antigen). The affinity of a molecule X for its partner Y can generally be represented by the dissociation constant (K_{D}). Affinity can be measured by common methods known in the art, including those described herein. Low-affinity antibodies generally bind antigens slowly and tend to dissociate readily, whereas high-affinity antibodies generally bind antigens faster and tend to remain bound longer. A variety of methods of measuring binding affinity are known in the art, any of which can be used for purposes of the present invention. Specific illustrative and exemplary embodiments for measuring binding affinity, i. e. binding strength are described in the following.

The term "K_{assoc}", "Kₐ" or "Kₒₙ", as used herein, are intended to refer to the association rate of a particular antibody-antigen interaction, whereas the term "K_{dis}", "K_{d}" or "K_{off}", as used herein, is intended to refer to the dissociation rate of a particular antibody-antigen interaction. In one embodiment, the term "K_{D}", as used herein, is intended to refer to the dissociation constant, which is obtained from the ratio of K_{d} to Kₐ (i. e. K_{d}/Kₐ) and is expressed as a molar concentration (M). The "K_{D}" or "K_{D} value" or "KD" or "KD value" according to this invention is in one embodiment measured by using surface plasmon resonance assays.

The expression "members of leukocyte immunoglobulin-like receptor subfamily A" or "LILRA" refers to leukocyte immunoglobulin-like receptor subfamily A, which, like the LILRBs, are a family of immunoreceptors that belong to leukocyte Ig-like receptors (LIRs) and that are expressed predominantly on monocytes and B cells and at lower levels on dendritic cells and natural killer (NK) cells. Examples are LILRA1, LILRA2, LILRA3, LILRA4, LILRA5 and LILRA6.

The expression "LILRB members other than LILRB2" refer to members of leukocyte immunoglobulin-like receptor subfamily B different from LILRB2. Examples are LILRB1, LILRB3, LILRB4 and LILRB5.

Affinity to recombinant human LILRB2 and recombinant cynomolgus LILRB2 was determined by surface plasmon resonance (SPR) measurements, as described in Example 1.7 (scFvs) and Example 2.3 (multispecific molecules).

Suitably, the LILRB2-BD of the present invention is further characterized by one or more of the following parameters:
a. when in scFv format, has a melting temperature (Tm), determined by differential scanning fluorimetry, in the range of 55°C to 85°C, particularly in the range of 60°C to 85°C, in particular wherein said antibodies are formulated in 50 mM phosphate-citrate buffer at pH 6.4, 150 mM NaCl;
b. when in scFv format, has a loss in monomer content, after storage for four weeks, at -80°C, of less than 5 %, less than 4 %, less than 3 %, less than 2 %, particularly less than 1.5 %, when said antibodies are at a starting concentration of 10 mg/ml, and in particular wherein said antibody is formulated in 50 mM phosphate citrate buffer with 150 mM NaCl at pH 6.4;
c. when in scFv format, has a loss in monomer content, after storage for four weeks, at 4°C, of less than 15 %, e.g. less than 12 %, less than 10 %, less than 9 %, less than 8 %, particularly less than 7 %, when said antibodies are at a starting concentration of 10 mg/ml, and in particular wherein said antibodies are formulated in 50 mM phosphate citrate buffer with 150 mM NaCl at pH 6.4;
d. when in scFv format, has a loss in monomer content, after storage for four weeks, at 40°C, of less than 15 %, e.g. less than 12 %, less than 10 %, less than 9 %, less than 8 %, particularly less than 7 %, when said antibodies are at a starting concentration of 10 mg/ml, and in particular wherein said antibodies are formulated in 50 mM phosphate citrate buffer with 150 mM NaCl at pH 6.4.

Differential scanning fluorimetry (DSF) measurements are performed as described earlier (Egan, et al., MAbs, 9(1) (2017), 68-84; Niesen, et al., Nature Protocols, 2(9) (2007) 2212-2221). In this DSF measurement procedure, the midpoint of transition for the thermal unfolding of the scFv constructs is determined by using the fluorescence dye SYPRO Orange (see Wong & Raleigh, Protein Science 25 (2016) 1834-1840). Typically, samples in phosphate-citrate buffer at pH 6.4 are prepared at a final protein concentration of 50 µg/ml and containing a final concentration of 5x SYPRO Orange in a total volume of 100 µl. Twenty-five microliters of prepared samples are added in triplicate to white-walled AB gene PCR plates. The assay is performed in a qPCR machine used as a thermal cycler, and the fluorescence emission is detected using the software's custom dye calibration routine. The PCR plate containing the test samples is subjected to a temperature ramp from 25°C to 96°C in increments of 1°C with 30 s pauses after each temperature increment. The total assay time is about 2 h. The Tm is calculated by the software GraphPad Prism using a mathematical second derivative method to calculate the inflection point of the curve. The reported Tm is typically an average of three measurements.

The loss in monomer content is as determined by area under the curve calculation of SE-HPLC chromatograms. SE-HPLC is a separation technique based on a solid stationary phase and a liquid mobile phase as outlined by the US Pharmacopeia (USP), chapter 621. This method separates molecules based on their size and shape utilizing a hydrophobic stationary phase and aqueous mobile phase. The separation of molecules is occurring between the void volume (V₀) and the total permeation volume (V_{T}) of a specific column. Measurements by SE-HPLC are performed on a Chromaster HPLC system (Hitachi High-Technologies Corporation) equipped with automated sample injection and a UV detector set to the detection wavelength of 280 nm. The equipment is controlled by the software EZChrom Elite (Agilent Technologies, Version 3.3.2 SP2) which also supports analysis of resulting chromatograms. Protein samples are cleared by centrifugation and kept at a temperature of 4-6°C in the autosampler prior to injection. For the analysis of scFv samples the column Shodex KW403-4F (Showa Denko Inc., #F6989202) is employed with a standardized buffered saline mobile phase (50 mM sodium-phosphate pH 6.5, 300 mM sodium chloride) at the recommended flow rate of 0.35 ml/min. The target sample load per injection was 5 µg. Samples are detected by an UV detector at a wavelength of 280 nm and the data recorded by a suitable software suite. The resulting chromatograms are analyzed in the range of V₀ to V_{T} thereby excluding matrix associated peaks with >10 min elution time.

Suitably, the LILRB2-BDs of the invention are binding domains provided in the present disclosure. The LILRB2-BDs of the invention include, but are not limited to, the humanized LILRB2-BDs whose sequences are listed in Table 1.

In a particular aspect, the present invention relates to An antibody binding domain, which specifically binds to LILRB2 (LILRB2-BD), comprising:
a) a variable heavy chain (VH),
   wherein the variable heavy chain comprises, from N-terminus to C-terminus, the regions HFR1-HCDR1-HFR2-HCDR2-HFR3-HCDR3-HFR4, wherein each HFR designates a heavy chain framework region, and each HCDR designates a heavy chain complementarity-determining region, and
b) a variable light chain (VL),
wherein the variable light chain comprises, from N-terminus to C-terminus, the regions LFR1-LCDR1-LFR2-LCDR2-LFR3-LCDR3-LFR4, wherein each LFR designates a light chain framework region, and each LCDR designates a light chain complementarity-determining region,
wherein
said HCDR1 has the sequence of SEQ ID NO: 1,
said HCDR2 has the sequences of SEQ ID NO: 2,
said HCDR3 has the sequence of SEQ ID NO: 3,
said LCDR1 has the sequence of SEQ ID NO: 4,
said LCDR2 has the sequence of SEQ ID NO: 6, and
said LCDR3 has the sequences of SEQ ID NO: 7;
or wherein
said HCDR1 has the sequence of SEQ ID NO: 1,
said HCDR2 has the sequences of SEQ ID NO: 2,
said HCDR3 has the sequence of SEQ ID NO: 3,
said LCDR1 has the sequence of SEQ ID NO: 4,
said LCDR2 has the sequence of SEQ ID NO: 5, and
said LCDR3 has the sequences of SEQ ID NO: 7;
or wherein
said HCDR1 has the sequence of SEQ ID NO: 20,
said HCDR2 has the sequences of SEQ ID NO: 21,
said HCDR3 has the sequence of SEQ ID NO: 22,
said LCDR1 has the sequence of SEQ ID NO: 23,
said LCDR2 has the sequence of SEQ ID NO: 25, and
said LCDR3 has the sequences of SEQ ID NO: 26;
or wherein
said HCDR1 has the sequence of SEQ ID NO: 36,
said HCDR2 has the sequences of SEQ ID NO: 37,
said HCDR3 has the sequence of SEQ ID NO: 38,
said LCDR1 has the sequence of SEQ ID NO: 39,
said LCDR2 has the sequence of SEQ ID NO: 41, and
said LCDR3 has the sequences of SEQ ID NO: 42;
particularly wherein
said HCDR1 has the sequence of SEQ ID NO: 1,
said HCDR2 has the sequence of SEQ ID NO: 2,
said HCDR3 has the sequence of SEQ ID NO: 3,
said LCDR1 has the sequence of SEQ ID NO: 4,
said LCDR2 has the sequence of SEQ ID NO: 5, and
said LCDR3 has the sequence of SEQ ID NO: 7;
or wherein
said HCDR1 has the sequence of SEQ ID NO: 1,
said HCDR2 has the sequence of SEQ ID NO: 2,
said HCDR3 has the sequence of SEQ ID NO: 3,
said LCDR1 has the sequence of SEQ ID NO: 4,
said LCDR2 has the sequence of SEQ ID NO: 6, and
said LCDR3 has the sequence of SEQ ID NO: 7;
more particularly wherein
said HCDR1 has the sequence of SEQ ID NO: 1,
said HCDR2 has the sequence of SEQ ID NO: 2,
said HCDR3 has the sequence of SEQ ID NO: 3,
said LCDR1 has the sequence of SEQ ID NO: 4,
said LCDR2 has the sequence of SEQ ID NO: 6, and
said LCDR3 has the sequence of SEQ ID NO: 7.

Other variable regions used in the invention include amino acid sequences that have been mutated, yet have at least 80, 85, 90, 91, 92, 93, 94, 95, 96, 97, 98 or 99 percent identity in the CDR regions with the CDR regions depicted in the sequences described in Table 1. Other variable regions used the invention include mutant amino acid sequences wherein no more than 1, 2, 3, 4 or 5, particularly 1 or 2, amino acids have been mutated in the CDR regions when compared with the CDR regions depicted in the sequence described in Table 1.

Suitably, the variable heavy chain regions, herein also referred to as VH domains, of the LILRB2-BDs of the invention, more particularly the heavy chain framework regions HFR1, HFR2, HFR3 and HFR4 of the LILRB2-BDs of the invention, belong to a VH3 or VH4 family, in particular to a VH3 family.

In the context of the present invention, the term "belonging to VHx family" or "selected from VHx family" means that the respective heavy chain framework sequence or sequences referred to, e. g. HFR1, HFR2, HFR3, HFR4, HFR1 to HFR3, HFR1 to HFR4, show the highest degree of homology to said VHx family. Likewise, the term "belonging to Vx family" or "selected from Vx family" means that the respective light chain framework sequence or sequences referred to, e. g. LFR1, LFR2, LFR3, LFR4, LFR1 to LFR3, LFR1 to LFR4, show the highest degree of homology to said Vx family. Examples of VH and VL families are given in Knappik et al., J. Mol. Biol. 296 (2000) 57-86, or in WO 2019/057787.

A specific example of a VH domain belonging to VH3 family is represented by SEQ ID NOs: 52 and 53, and a specific example of a VH domain belonging to VH4 family is represented by SEQ ID NO: 56. Particularly, a VH belonging to VH3 family, as used herein, is a VH, wherein each HFR1 to HFR4, independently of each other, have at least 85 %, particularly at least 90 %, more particularly at least 95 % sequence identity to the corresponding HFR1 to HFR4 of SEQ ID NO: 52 or 53. In particular, heavy chain framework regions HFR1 to HFR4 are selected from SEQ ID NOs: 52 and 53 (Table 2, regions marked in non-bold). Alternative examples of VH3 and VH4 sequences, and examples of other VHx sequences, may be found in Knappik et al., J. Mol. Biol. 296 (2000) 57-86 or in WO 2019/057787.

Suitably, the variable light chain regions, herein also referred to as VL domains, of the LILRB2-BDs of the invention comprise Vκ frameworks LFR1 to LFR4, particularly Vκ1 or Vκ3 frameworks LFR1 to LFR4, particularly Vκ1 frameworks LFR1 to LFR4. In the case where said LILRB2-BDs are not in a Fab format, for example where said LILRB-BDs are in an scFv-format, said LILRB2-BDs comprise: Vκ frameworks LFR1 to LFR3, particularly Vκ1 or Vκ3 frameworks LFR1 to LFR3, particularly Vκ1 frameworks LFR1 to LFR3, and a framework LFR4, which is selected from a Vλ LFR4.

A specific example of a VL having Vκ1 frameworks LFR1 to LFR4 is represented by SEQ ID NO: 59 (Table 2, FR regions are marked in non-bold). Suitable Vκ1 frameworks LFR1 to LFR3 with an exemplary Vλ LFR4 are set forth in SEQ ID NOs: 60 and 61 (Table 2, FR regions are marked in non-bold). Alternative examples of Vκ1 sequences, and examples of Vκ2, Vκ3 or Vκ4 sequences, may be found in Knappik et al., J. Mol. Biol. 296 (2000) 57-86. Suitable Vκ1 frameworks LFR1 to LFR3 or LFR1 to LFR4 also comprise, independently of each other, the amino acid sequences having at least 70, 80, 90 percent identity to each of the amino acid sequences corresponding to LFR1 to LFR3 or LFR1 to LFR4 of SEQ ID NO: 59 (Table 2, FR regions are marked in non-bold). Suitable Vλ LFR4 are as set forth in SEQ ID NO: 62 to SEQ ID NO: 69 and in SEQ ID NO: 70 comprising a single cysteine residue, particular in a case where a second single cysteine is present in the corresponding VH chain, particularly in position 51 (AHo numbering) of VH, for the formation of an interdomain disulfide bond. In one embodiment, the VL domains of the binding domains of the multispecific antibodies of the invention, when being in Fv or scFv-format, comprises Vλ LFR4 comprising the amino acid sequence having at least 80, particularly 90, percent identity to an amino acid sequence selected from any of SEQ ID NO: 62 to SEQ ID NO: 70, or in other words having 1 or 2 substitutions when compared to the closest amino acid sequence selected from any of SEQ ID NO: 62 to SEQ ID NO: 70. Particularly, the multispecific antibodies of the invention, when being in Fv or scFv-format, comprises Vλ LFR4 comprising an amino acid sequence selected from SEQ ID NOs: 62 or 70.

The LILRB2-BDs of the invention comprise a VH domain listed in Tables 1 and 2. Suitably, the LILRB2-BDs of the invention comprise a VH amino acid sequence listed in Tables 1 and 2, wherein no more than 10 amino acids, particularly no more than 5 amino acids, particularly no more than 4 amino acids, particularly no more than 3 amino acids, particularly no more than 2 amino acids, particularly no more than 1 amino acid, in the framework sequences (i. e., the sequences which are not CDR sequences) have been mutated (wherein a mutation is, as various non-limiting examples, an addition, substitution or deletion). Other LILRB2-BDs of the invention include amino acids that have been mutated, yet have at least 90, 91, 92, 93, 94, 95, 96, 97, 98 or 99 percent identity in the VH regions with the VH regions depicted in the corresponding sequences described in Tables 1 and 2, including VH domains comprising at least positions 5 to 140 (AHo numbering), particularly at least positions 3 to 145 of one of the sequences shown in Tables 1 and 2.

In particular, the LILRB2-BDs of the invention comprise a VL domain listed in Tables 1 and 2. Suitably, the LILRB2-BDs of the invention comprise a VL amino acid sequence listed in Tables 1 and 2, wherein no more than 10 amino acids, particularly no more than 5 amino acids, particularly no more than 4 amino acids, particularly no more than 3 amino acids, particularly no more than 2 amino acids, particularly no more than 1 amino acid, in the framework sequences (i. e., the sequences which are not CDR sequences) have been mutated (wherein a mutation is, as various non-limiting examples, an addition, substitution or deletion). Other LILRB2-BDs of the invention include amino acids that have been mutated, yet have at least 90, 91, 92, 93, 94, 95, 96, 97, 98 or 99 percent identity in the VL regions with a VL region depicted in the sequences described in Tables 1 and 2, including VL domains comprising at least positions 5 to 140 (AHo numbering), particularly at least positions 3 to 145 of one of the sequences shown in Tables 1 and 2.

In particular embodiments, the LILRB2-BDs of the present invention are scFv antibody fragments. Specific but non-limiting examples of the LILRB2-BDs of the present invention are the scFv antibody fragments having a sequence selected from SEQ ID NOs: 16, 17, 18, 19, 33, 34, 35, 49, 50 and 51.

In a further aspect, the present invention relates to a monospecific antibody comprising one or two, preferably two, LILRB2-BDs as defined herein (anti-LILRB2 antibodies). Specific but non-limiting examples of such monospecific anti-LILRB2 antibodies are:
- PRO3603, having the heavy and light chain sequences as defined in SEQ ID NOs: 75 and 76;
- PRO3635, having the heavy and light chain sequences as defined in SEQ ID NOs: 77 and 78; and
- PRO4710, having the heavy and light chain sequences as defined in SEQ ID NOs: 79 and 80.

In yet another aspect, the present invention relates to a multispecific antibody comprising:
a) one or two LILRB2-BDs as defined herein;
b) at least one binding domain, which specifically binds to a target different from LILRB2.

In some embodiments, the multispecific antibody does not comprise an immunoglobulin Fc region. In these embodiments, the multispecific antibody is preferably in a format selected from the group consisting of: a tandem scDb (Tandab), a linear dimeric scDb (LD-scDb), a circular dimeric scDb (CD-scDb), a tandem tri-scFv, a tribody (Fab-(scFv)2), a Fab-Fv2, a triabody, an scDb-scFv, a tetrabody, a didiabody, a tandem-di-scFv and a MATCH.

In some other embodiments, the multispecific antibody comprises an immunoglobulin Fc region.

In particular embodiments, the immunoglobulin Fc region comprised in the multispecific antibodies of the invention is an active Fc region.

In further embodiments, the immunoglobulin Fc region comprised in the multispecific antibodies of the invention may additionally or alternatively have one or more amino acid modifications selected from the group consisting of:
a) modifications to enhance heterodimerization of heavy chains, such as the knob-into-hole (KiH) technology;
b) modifications that increase or decrease, in particular increase, Fc receptor binding, when compared to the respective unmodified immunoglobulin Fc region thereof;
c) modifications that increase serum half-life, when compared to the respective unmodified immunoglobulin Fc region thereof.

In particular embodiments, the immunoglobulin Fc region comprised in the multispecific antibodies of the invention is selected from an Fc region of an IgG subclass, particularly an immunoglobulin Fc region of human IgG1, IgG2 (IgG2A, IgG2B), IgG3 or IgG4, particularly of human IgG1 or IgG2, particularly of human IgG1.

Specific but non-limiting examples of multispecific antibodies, where the LILRB2-BDs of the invention have successfully been incorporated, are the anti-CD47xPDL1xLILRB2 antibodies PRO4672, PRO4687, PRO5052 and PRO5055 whose sequences are listed in Table 4.

Suitably, the binding domains of the multispecific antibodies of the invention are operably linked. The binding domains of the multispecific antibodies of the invention are capable of binding to their respective antigens or receptors simultaneously. The term "simultaneously", as used herein refers for example to the simultaneous binding of the CD47-BD and the PDL1-BD or the simultaneous binding of the PDL1-BD and the LILRB2-BD.

The term "operably linked", as used herein, indicates that two molecules (e. *g.,* polypeptides, domains, binding domains) are attached in a way that each molecule retains functional activity. Two molecules can be "operably linked" whether they are attached directly or indirectly (e. g., via a linker, via a moiety, via a linker to a moiety).

The term "linker" refers to a peptide or other moiety that is optionally located between binding domains or antibody fragments used in the invention. A number of strategies may be used to covalently link molecules together. These include, but are not limited to, polypeptide linkages between N- and C-termini of proteins or protein domains, linkage via disulfide bonds, and linkage via chemical cross-linking reagents. In one aspect of this embodiment, the linker is a peptide bond, generated by recombinant techniques or peptide synthesis. Choosing a suitable linker for a specific case where two polypeptide chains are to be connected depends on various parameters, including but not limited to the nature of the two polypeptide chains (e. g., whether they naturally oligomerize), the distance between the N- and the C-termini to be connected if known, and/or the stability of the linker towards proteolysis and oxidation. Furthermore, the linker may contain amino acid residues that provide flexibility.

In the context of the present invention, the term "polypeptide linker" refers to a linker consisting of a chain of amino acid residues linked by peptide bonds that is connecting two domains, each being attached to one end of the linker. The polypeptide linker should have a length that is adequate to link two molecules in such a way that they assume the correct conformation relative to one another so that they retain the desired activity. In particular embodiments, the polypeptide linker has a continuous chain of between 2 and 30 amino acid residues (e. *g.,* 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acid residues). In addition, the amino acid residues selected for inclusion in the polypeptide linker should exhibit properties that do not interfere significantly with the activity of the polypeptide. Thus, the linker peptide on the whole should not exhibit a charge that would be inconsistent with the activity of the polypeptide, or interfere with internal folding, or form bonds or other interactions with amino acid residues in one or more of the monomers that would seriously impede the binding of receptor monomer domains. In particular embodiments, the polypeptide linker is non-structured polypeptide. Useful linkers include glycine-serine, or GS linkers. By "Gly-Ser" or "GS" linkers is meant a polymer of glycines and serines in series (including, for example, (Gly-Ser)ₙ, (GSGGS)ₙ (SEQ ID NO: 91), (GGGGS)ₙ (SEQ ID NO: 92) and (GGGS)ₙ (SEQ ID NO: 93), where n is an integer of at least one), glycine-alanine polymers, alanine-serine polymers, and other flexible linkers such as the tether for the shaker potassium channel, and a large variety of other flexible linkers, as will be appreciated by those in the art. Glycine-serine polymers are preferred since oligopeptides comprising these amino acids are relatively unstructured, and therefore may be able to serve as a neutral tether between components. Secondly, serine is hydrophilic and therefore able to solubilize what could be a globular glycine chain. Third, similar chains have been shown to be effective in joining subunits of recombinant proteins such as single-chain antibodies.

In particular embodiments the one or two LILRB2-BD(s) is/are fused to the heavy chain of the immunoglobulin Fc region via a linker L1.

The linker L1 is a peptide of 2-30 amino acids, more particularly 5-25 amino acids, and most particularly 10-20 amino acids. In particular embodiments, said linker L1 comprises one or more units of four (4) glycine amino acid residues and one (1) serine amino acid residue (GGGGS)ₙ (SEQ ID NO:71), wherein n=1, 2, 3, 4 or 5, particularly n=2.

The scFv domains of the multispecific antibodies of the invention comprise a variable heavy chain domain (VH) and a variable light chain domain (VL) connected by a linker L2.

The linker L2 is a peptide of 10-40 amino acids, more particularly 15-30 amino acids, and most particularly 20-25 amino acids. In particular embodiments, said linker L2 comprises one or more units of four (4) glycine amino acid residues and one (1) serine amino acid residue (GGGGS)ₙ (SEQ ID NO:73), wherein n=1, 2, 3, 4, 5, 6, 7 or 8, particularly n=4.

The antibodies or multispecific antibodies of the invention can be produced using any convenient antibody-manufacturing method known in the art (see, e. g., Fischer, N. & Leger, O., Pathobiology 74 (2007) 3-14 with regard to the production of bispecific constructs; Hornig, N. & Färber-Schwarz, A., Methods Mol. Biol. 907 (2012)713-727, and WO 99/57150 with regard to bispecific diabodies and tandem scFvs). Specific examples of suitable methods for the preparation of the bispecific construct further include, inter alia, the Genmab (see Labrijn et al., Proc. Natl. Acad. Sci. USA 110 (2013) 5145-5150) and Merus (see de Kruif et al., Biotechnol. Bioeng. 106 (2010) 741-750) technologies. Methods for production of bispecific antibodies comprising a functional or active antibody Fc part are also known in the art (see, e. g., Zhu et al., Cancer Lett. 86 (1994) 127-134); and Suresh et al., Methods Enzymol. 121 (1986) 210-228).

These methods typically involve the generation of monoclonal antibodies, for example by means of fusing myeloma cells with the spleen cells from a mouse that has been immunized with the desired antigen using the hybridoma technology (see, e. g., Yokoyama et al., Curr. Protoc. Immunol. Chapter 2, Unit 2.5, 2006) or by means of recombinant antibody engineering (repertoire cloning or phage display/yeast display) (see, e. g., Chames & Baty, FEMS Microbiol. Letters 189 (2000) 1-8), and the combination of the antigen-binding domains or fragments or parts thereof of two or more different monoclonal antibodies to give a bispecific or multispecific construct using known molecular cloning techniques.

The multispecific antibodies of the invention can be prepared by conjugating the constituent binding specificities, using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-5-acetyl-thioacetate (SATA), 5,5'-dithiobis (2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl)-cyclohexane-I-carboxylate (sulfo-SMCC) (see e. g., Karpovsky et al., 1984 J. Exp. Med. 160: 1686; Liu, M A et al., 1985 Proc. Natl. Acad. Sci. USA 82:8648). Other methods include those described in Paulus, 1985 Behring Ins. Mitt. No. 78, 118-132; Brennan et al., 1985 Science 229:81-83, and Glennie et al., 1987 J. Immunol. 139: 2367-2375. Conjugating agents are SATA and sulfo-SMCC, both available from Pierce Chemical Co. (Rockford, III).

When the binding specificities are antibodies, they can be conjugated by sulfhydryl bonding of the C-terminus hinge regions of the two heavy chains. In a particular embodiment, the hinge region is modified to contain an odd number of sulfhydryl residues, for example one, prior to conjugation.

Alternatively, two or more binding specificities can be encoded in the same vector and expressed and assembled in the same host cell. This method is particularly useful where the bispecific molecule is a mAb x Fab, a mAb x scFv, a mAb x dsFv or a mAb x Fv fusion protein. Methods for preparing multispecific antibodies and molecules are described for example in U.S. Pat. No. 5,260,203; U.S. Pat. No. 5,455,030; U.S. Pat. No. 4,881,175; U.S. Pat. No. 5,132,405; U.S. Pat. No. 5,091,513; U.S. Pat. No. 5,476,786; U.S. Pat. No. 5,013,653; U.S. Pat. No. 5,258,498; and U.S. Pat. No. 5,482,858.

Binding of the multispecific antibodies to their specific targets can be confirmed by, for example, enzyme-linked immunosorbent assay (ELISA), radioimmunoassay (REA), FACS analysis, bioassay (e. g., growth inhibition), or Western Blot assay. Each of these assays generally detects the presence of protein-antibody complexes of particular interest by employing a labeled reagent (e. *g.,* an antibody) specific for the complex of interest.

In a further aspect, the invention provides a nucleic acid or two nucleic acids or three nucleic acids encoding the multispecific antibody of the invention. Such nucleic acids can be optimized for expression in mammalian cells.

The term "nucleic acid" is used herein interchangeably with the term "polynucleotide(s)" and refers to one or more deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphorates, 2-O-methyl ribonucleotides, peptide-nucleic acids (PNAs). Unless otherwise indicated, a particular nucleic acid sequence also implicitly encompasses conservatively modified variants thereof (e. g., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., Nucleic Acid Res. 19:5081, 1991; Ohtsuka et al., J. Biol. Chem. 260:2605-2608, 1985; and Rossolini et al., Mol. Cell. Probes 8:91-98, 1994).

The invention provides substantially purified nucleic acid molecules which encode polypeptides comprising segments or domains of the multispecific antibody described above. When expressed from appropriate expression vectors, polypeptides encoded by these nucleic acid molecules are capable of exhibiting antigen-binding capacities of the multispecific antibody of the present invention.

The polynucleotide sequences can be produced by *de novo* solid-phase DNA synthesis or by PCR mutagenesis of an existing sequence (e. g., sequences as described in the Examples below) encoding the multispecific antibody of the invention or fragments thereof or binding domains thereof. Direct chemical synthesis of nucleic acids can be accomplished by methods known in the art, such as the phosphotriester method of Narang et al., 1979, Meth. Enzymol. 68:90; the phosphodiester method of Brown et al., Meth. Enzymol. 68: 109, 1979; the diethylphosphoramidite method of Beaucage et al., Tetra. Lett., 22: 1859, 1981; and the solid support method of U.S. Pat. No. 4,458,066. Introducing mutations to a polynucleotide sequence by PCR can be performed as described in, e. *g.,* PCR Technology: Principles and Applications for DNA Amplification, H. A. Erlich (Ed.), Freeman Press, NY, N.Y., 1992; PCR Protocols: A Guide to Methods and Applications, Innis et al. (Ed.), Academic Press, San Diego, Calif, 1990; Mattila et al., Nucleic Acids Res. 19:967, 1991; and Eckert et al., PCR Methods and Applications 1:17, 1991.

Also provided in the invention are expression vectors and host cells for producing the multispecific antibody of the invention or fragments thereof or binding domains thereof.

The term "vector" is intended to refer to a polynucleotide molecule capable of transporting another polynucleotide to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e. g., bacterial vectors having a bacterial origin of replication and episomal mammalian vectors). Other vectors (e. g., non-episomal mammalian vectors) can be integrated into the genome of a host cell upon introduction into the host cell, and thereby are replicated along with the host genome.

Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (e. g., replication defective retroviruses, adenoviruses and adeno- associated viruses), which serve equivalent functions. In this particular context, the term "operably linked" refers to a functional relationship between two or more polynucleotide (e. g., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, i. e., they are cis-acting. However, some transcriptional regulatory sequences, such as enhancers, need not be physically contiguous or located in close proximity to the coding sequences whose transcription they enhance.

Various expression vectors can be employed to express the polynucleotides encoding the multispecific antibody chains. Both viral-based and non-viral expression vectors can be used to produce the antibodies in a mammalian host cell. Non-viral vectors and systems include plasmids, episomal vectors, typically with an expression cassette for expressing a protein or RNA, and human artificial chromosomes (see, e. g., Harrington et al., Nat Genet. 15:345, 1997). For example, non-viral vectors useful for expression of the polynucleotides encoding the multispecific antibody chains in mammalian (e. g., human) cells include pThioHis A, B and C, pcDNA3.1/His, pEBVHis A, B and C, (Invitrogen, San Diego, Calif.), MPS V vectors, and numerous other vectors known in the art for expressing other proteins. Useful viral vectors include vectors based on retroviruses, adenoviruses, adeno-associated viruses, herpes viruses, vectors based on SV40, papilloma virus, HBP Epstein Barr virus, vaccinia virus vectors and Semliki Forest virus (SFV). See, Brent et al., supra; Smith, Annu. Rev. Microbiol. 49:807, 1995; and Rosenfeld et al., Cell 68: 143, 1992.

The choice of expression vector depends on the intended host cells in which the vector is to be expressed. Typically, the expression vectors contain a promoter and other regulatory sequences (e. g., enhancers) that are operably linked to the polynucleotides encoding a multispecific antibody chain or a fragment. In one embodiment, an inducible promoter is employed to prevent expression of inserted sequences except under inducing conditions. Inducible promoters include, e. g., arabinose, lacZ, metallothionein promoter or a heat shock promoter. Cultures of transformed organisms can be expanded under non-inducing conditions without biasing the population for coding sequences whose expression products are better tolerated by the host cells. In addition to promoters, other regulatory elements may also be required or desired for efficient expression of a multispecific antibody chain or a fragment. These elements typically include an ATG initiation codon and adjacent ribosome binding site or other sequences. In addition, the efficiency of expression may be enhanced by the inclusion of enhancers appropriate to the cell system in use (see, e. *g.,* Scharf et al., Results Probl. Cell Differ. 20: 125, 1994; and Bittner et al., Meth. Enzymol., 153:516, 1987). For example, the SV40 enhancer or CMV enhancer may be used to increase expression in mammalian host cells.

Vectors to be used typically encode the multispecific antibody light and heavy chain or chains including constant regions or parts thereof. Such vectors allow expression of the variable regions as fusion proteins with the constant regions thereby leading to production of intact antibodies and antigen-binding fragments thereof. Typically, such constant regions are human.

The term "recombinant host cell" (or simply "host cell") refers to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The host cells for harboring and expressing the multispecific antibody of the invention can be either prokaryotic or eukaryotic. *E. coli* is one prokaryotic host useful for cloning and expressing the polynucleotides of the present invention. Other microbial hosts suitable for use include bacilli, such as *Bacillus subtilis,* and other enterobacteriaceae, such as Salmonella, Serratia, and various Pseudomonas species. In these prokaryotic hosts, one can also make expression vectors, which typically contain expression control sequences compatible with the host cell (e. *g.,* an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express the multispecific antibodies of the invention. Insect cells in combination with baculovirus vectors can also be used.

In one embodiment, mammalian host cells are used to express and produce the multispecific antibody of the invention. For example, they can be either a hybridoma cell line expressing endogenous immunoglobulin genes or a mammalian cell line harboring an exogenous expression vector. These include any normal mortal or normal or abnormal immortal animal or human cell. For example, a number of suitable host cell lines capable of secreting intact immunoglobulins have been developed including the CHO cell lines, various COS cell lines, HeLa cells, myeloma cell lines, transformed B-cells and hybridomas. The use of mammalian tissue cell culture to express polypeptides is discussed generally in, e. *g.,* Winnacker, FROM GENES TO CLONES, VCH Publishers, N.Y., N.Y., 1987. Expression vectors for mammalian host cells can include expression control sequences, such as an origin of replication, a promoter, and an enhancer (see, e. g., Queen, et al., Immunol. Rev. 89:49-68, 1986), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage- specific, and/or modulatable or regulatable. Useful promoters include, but are not limited to, the metallothionein promoter, the constitutive adenovirus major late promoter, the dexamethasone-inducible MMTV promoter, the SV40 promoter, the MRP pol III promoter, the constitutive MPS V promoter, the tetracycline-inducible CMV promoter (such as the human immediate-early CMV promoter), the constitutive CMV promoter, and promoter-enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Green, M. R., and Sambrook, J., Molecular Cloning: A Laboratory Manual (Fourth Edition), Cold Spring Harbor Laboratory Press (2012)). Other methods include, e. g., electroporation, calcium phosphate treatment, liposome-mediated transformation, injection and microinjection, ballistic methods, virosomes, immunoliposomes, polycation-nucleic acid conjugates, naked DNA, artificial virions, fusion to the herpes virus structural protein VP22 (Elliot and O'Hare, Cell 88:223, 1997), agent-enhanced uptake of DNA, and ex *vivo* transduction. For long-term, high-yield production of recombinant proteins, stable expression will often be desired. For example, cell lines which stably express the multispecific antibody of the invention can be prepared using expression vectors of the invention which contain viral origins of replication or endogenous expression elements and a selectable marker gene. Following the introduction of the vector, cells may be allowed to grow for 1 to 2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type. The present invention thus provides a method of producing the antibody of the invention or antigen-binding fragment thereof, wherein said method comprises the step of culturing a host cell comprising a nucleic acid or a vector encoding the antibody of the invention or antigen-binding fragment thereof, whereby said antibody of the disclosure or a fragment thereof is expressed.

In one further aspect, the present invention relates to a method of producing the antibody binding domain, the antibody or the multispecific antibody of the invention, the method comprising the step of culturing a host cell expressing a nucleic acid or two nucleic acids or three nucleic acids encoding the antibody binding domain, the antibody or the multispecific antibody of the invention. In particular, the present invention relates to a method of producing the antibody binding domain, the antibody or multispecific antibody of the invention, the method comprising (i) providing a nucleic acid or two nucleic acids or three nucleic acids encoding the antibody binding domain, the antibody or multispecific antibody of the invention or a vector or two vectors or three vectors encoding the antibody binding domain, the antibody or multispecific antibody of the invention, expressing said nucleic acid or nucleic acids, or said vector or vectors, and collecting said antibody binding domain, said antibody or said multispecific antibody from the expression system, or (ii) providing a host cell or host cells expressing a nucleic acid or nucleic acids encoding the antibody binding domain, the antibody or multispecific antibody of the invention, culturing said host cell or said host cells; and collecting said antibody binding domain, said antibody or said multispecific antibody from the cell culture.

In a further aspect, the present invention relates to a pharmaceutical composition comprising the antibody or the multispecific antibody of the invention, and a pharmaceutically acceptable carrier. "Pharmaceutically acceptable carrier" means a medium or diluent that does not interfere with the structure of the antibodies. Pharmaceutically acceptable carriers enhance or stabilize the composition, or facilitate preparation of the composition. Pharmaceutically acceptable carriers include solvents, dispersion media, coatings, antibacterial and antifungal agents, isotonic and absorption delaying agents, and the like that are physiologically compatible.

Certain, of such carriers enable pharmaceutical compositions to be formulated as, for example, tablets, pills, dragees, capsules, liquids, gels, syrups, slurries, suspension and lozenges for the oral ingestion by a subject. Certain of such carriers enable pharmaceutical compositions to be formulated for injection, infusion or topical administration. For example, a pharmaceutically acceptable carrier can be a sterile aqueous solution.

The pharmaceutical composition of the invention can be administered by a variety of methods known in the art. The route and/or mode of administration vary depending upon the desired results. Administration can be intravenous, intramuscular, intraperitoneal, or subcutaneous, or administered proximal to the site of the target. In particular embodiments, the administration is intramuscular, or subcutaneous, particularly subcutaneous. The pharmaceutically acceptable carrier should be suitable for intravenous, intramuscular, subcutaneous, parenteral, spinal or epidermal administration (e. *g.,* by injection or infusion), particularly for intramuscular or subcutaneous administration. Depending on the route of administration, the active compound, i. e., the multispecific antibody of the invention, may be coated in a material to protect the compound from the action of acids and other natural conditions that may inactivate the compound.

The pharmaceutical compositions of the invention can be prepared in accordance with methods well known and routinely practiced in the art. See, e. g., Remington: The Science and Practice of Pharmacy, Mack Publishing Co., 20th ed., 2000; and Sustained and Controlled Release Drug Delivery Systems, J. R. Robinson, ed., Marcel Dekker, Inc., New York, 1978. Pharmaceutical compositions are preferably manufactured under GMP conditions. Typically, a therapeutically effective dose or efficacious dose of the multispecific antibody of the invention is employed in the pharmaceutical compositions of the invention. The multispecific antibodies of the invention are formulated into pharmaceutically acceptable dosage forms by conventional methods known to those of skill in the art. Dosage regimens are adjusted to provide the optimum desired response (e. *g.,* a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions of the invention can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

The antibody or multispecific antibody of the invention is usually administered on multiple occasions. Intervals between single dosages can be weekly, monthly or yearly. Intervals can also be irregular as indicated by measuring blood levels of the multispecific antibody of the invention in the patient. Alternatively, the antibody or multispecific antibody of the invention can be administered as a sustained release formulation, in which case less frequent administration is required. Dosage and frequency vary depending on the half-life of the antibody in the patient. In general, humanized antibodies show longer half-life than that of chimeric antibodies and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

In one aspect, the present invention relates to the antibody or multispecific antibody of the invention or the pharmaceutical composition of the invention for use as a medicament. In a suitable embodiment, the present invention provides the antibody or multispecific antibody or the pharmaceutical composition for use in the treatment of a disease, particularly a human disease, more particularly a human disease selected from cancer.

In another aspect, the present invention provides the pharmaceutical composition for use in the manufacture of a medicament for the treatment of a disease, particularly a human disease, more particularly a human disease selected from cancer.

In another aspect, the present invention relates to the use of the antibody or the multispecific antibody or the pharmaceutical composition of the present invention for treating a disease, particularly a human disease, more particularly a human disease selected from cancer, in a subject in need thereof.

In another aspect, the present invention relates to a method of treating a subject comprising administering to the subject a therapeutically effective amount of the antibody or the multispecific antibody or the pharmaceutical composition of the present invention. In a suitable embodiment, the present invention relates to a method for the treatment of a disease, particularly a human disease, more particularly a human disease selected from cancer, in a subject comprising administering to the subject a therapeutically effective amount of the multispecific antibody of the present invention.

The term "subject" includes human and non-human animals.

The term "animals" include all vertebrates, e. g., non-human mammals and non-mammals, such as non-human primates, sheep, dog, cow, chickens, amphibians, and reptiles. Except when noted, the terms "patient" or "subject" are used herein interchangeably.

The terms "treatment", "treating", "treat", "treated", and the like, as used herein, refer to obtaining a desired pharmacologic and/or physiologic effect. The effect may be therapeutic in terms of a partial or complete cure for a disease and/or adverse effect attributable to the disease or delaying the disease progression. "Treatment", as used herein, covers any treatment of a disease in a mammal, e. *g.,* in a human, and includes: (a) inhibiting the disease, i. e., arresting its development; and (b) relieving the disease, i. e., causing regression of the disease.

The term "therapeutically effective amount" or "efficacious amount" refers to the amount of an agent that, when administered to a mammal or other subject for treating a disease, is sufficient to affect such treatment for the disease. The "therapeutically effective amount" will vary depending on the agent, the disease and its severity and the age, weight, etc., of the subject to be treated.

In one embodiment, the disease is a cancer selected from: a benign or especially malignant tumor, solid tumors, mesothelioma, brain cancer, kidney cancer, liver cancer, adrenal gland cancer, bladder cancer, breast cancer, stomach cancer (e. g., gastric tumors), esophageal cancer, ovarian cancer, cervical cancer, thymic cancer, choriocarcinoma, oral cancer, salivary cancer, colon cancer, rectum cancer, prostate cancer, pancreatic cancer, lung cancer (e. g., non-small cell lung cancer and small cell lung cancer), vaginal cancer, thyroid cancer, melanoma (e. g., unresectable or metastatic melanoma), renal cell carcinoma, sarcoma, glioma, glioblastoma, multiple myeloma or gastrointestinal cancer, especially colon carcinoma or colorectal adenoma, a tumor of the neck and head, endometrial cancer, Cowden syndrome, Lhermitte-Duclos disease, Bannayan-Zonana syndrome, prostate hyperplasia, a neoplasia, especially of epithelial character, preferably mammary carcinoma or squamous cell carcinoma, chronic lymphocytic leukemia, chronic myelogenous leukemia (e. g., Philadelphia chromosome-positive chronic myelogenous leukemia), acute lymphoblastic leukemia (e. g., Philadelphia chromosome-positive acute lymphoblastic leukemia), non-Hodgkin's lymphoma, plasma cell myeloma, Hodgkin's lymphoma, a leukemia, and any combination thereof.

### Sequence listing (mutations designated according to AHo numbering scheme; the CDRs defined according to Numab CDR definition, unless specified otherwise)

**Table 1. Examples of LILRB2 binding domains according to the present invention CDR residues are shown in bold and italic letters.**

| **Anti-LILRB2 antibody variable domains** | | |
|---|---|---|
| *68-18-E11* - *based sequences* | | |
| 1 | **HCDR1** (H27-H42; AHo numbering) | ***GFSFTNNYYMC*** |
| 2 | **HCDR2** (H57-H76; AHo numbering) | ***CMLVGAWGNTYYAS*WANG** |
| 3 | **HCDR3** (H108-H138; AHo numbering) | ***RARDYTWTLGGDPETSGFNL*** |
| 4 | **LCDR1** (L24-L42; AHo numbering) | ***QASQSIGSNLA*** |
| 5 | **LCDR2** (L58-L72; AHo numbering) | ***KASTLES*** |
| 6 | LCDR2 (L58-L72; AHo numbering) | ***KGGTLES*** |
| 7 | **LCDR3** (L107-L138; AHo numbering) | ***QSTAGSSRSGNYGYV*** |
| 8 | **VH** 68-18-E11-sc07 | QQLVESGGGLVQPGGSLRLSCAASGFSFTNNYYMCWVRQAPGKGLEWIACMLVG AWGNTYYASWANGRFTISKDNSKNTVYLQMNSLRAEDTAVYFCARARDYTWTLG GDPETSGFNLWGQGTLVTVSS |
| 9 | **VH** 68-18-E11-sc07_(T101S_T146K) | QQLVESGGGLVQPGGSLRLSCAASGFSFTNNYYMCWVRQAPGKGLEWIACMLVG AWGNTYYASWANGRFTISKDNSKNTVYLQMNSLRAEDSAVYFCARARDYTWTLG GDPETSGFNLWGQGTLVKVSS |
| 10 | **VH** 68-18-E11-sc07_(G51C_T101S_T146K) | QQLVESGGGLVQPGGSLRLSCAASGFSFTNNYYMCWVRQAPGKCLEWIACMLVG AWGNTYYASWANGRFTISKDNSKNTVYLQMNSLRAEDSAVYFCARARDYTWTLG GDPETSGFNLWGQGTLVKVSS |
| 11 | **VL** 68-18-E11-sc07 | DVQMTQSPSSLSASVGDRVTITCQASQSIGSNLAWYQQKPGKPPKLLIYKASTLES GVPSRFSGSGSGTDFTLTISSLQPEDFATYYCQSTAGSSRSGNYGYVFGTGTKVTV LG |
| 12 | **VL** 68-18-E11-sc07_(A67G_S68G) | DVQMTQSPSSLSASVGDRVTITCQASQSIGSNLAWYQQKPGKPPKLLIYKGGTLES GVPSRFSGSGSGTDFTLTISSLQPEDFATYYCQSTAGSSRSGNYGYVFGTGTKVTV LG |
| 13 | **VL** 68-18-E11-sc07_(A67G_S68G T141C) | DVQMTQSPSSLSASVGDRVTITCQASQSIGSNLAWYQQKPGKPPKLLIYKGGTLES GVPSRFSGSGSGTDFTLTISSLQPEDFATYYCQSTAGSSRSGNYGYVFGCGTKVT VLG |
| 14 | **VL** 68-18-E11-sc07_(KappaCap) | DVQMTQSPSSLSASVGDRVTITCQASQSIGSNLAWYQQKPGKPPKLLIYKASTLES GVPSRFSGSGSGTDFTLTISSLQPEDFATYYCQSTAGSSRSGNYGYVFGQGTKVEI K |
| 15 | **VL** 68-18-E11-sc07_(A67G S68G_KappaCap) | DVQMTQSPSSLSASVGDRVTITCQASQSIGSNLAWYQQKPGKPPKLLIYKGGTLES GVPSRFSGSGSGTDFTLTISSLQPEDFATYYCQSTAGSSRSGNYGYVFGQGTKVEI K |
| 16 | **scFv** (VL-linker-VH) 68-18-E11-sc07 | DVQMTQSPSSLSASVGDRVTITCQASQSIGSNLAWYQQKPGKPPKLLIYKASTLES GVPSRFSGSGSGTDFTLTISSLQPEDFATYYCQSTAGSSRSGNYGYVFGTGTKVTV LGGGGGSGGGGSGGGGSGGGGSQQLVESGGGLVQPGGSLRLSCAASGFSFTN NYYMCWVRQAPGKGLEWIACMLVGAWGNTYYASWANGRFTISKDNSKNTVYLQ MNSLRAEDTAVYFCARARDYTWTLGGDPETSGFNLWGQGTLVTVSS |
| 17 | **scFv** (VL-linker-VH) 68-18-E11-sc07_(VL _A67G_S68G) | DVQMTQSPSSLSASVGDRVTITCQASQSIGSNLAWYQQKPGKPPKLLIYKGGTLES GVPSRFSGSGSGTDFTLTISSLQPEDFATYYCQSTAGSSRSGNYGYVFGTGTKVTV LGGGGGSGGGGSGGGGSGGGGSQQLVESGGGLVQPGGSLRLSCAASGFSFTN NYYMCWVRQAPGKGLEWIACMLVGAWGNTYYASWANGRFTISKDNSKNTVYLQ MNSLRAEDTAVYFCARARDYTWTLGGDPETSGFNLWGQGTLVTVSS |
| 18 | **scFv** (VL-linker-VH) 68-18-E11-sc07_(VL _A67G_S68G; VH_T101S_T146K) | DVQMTQSPSSLSASVGDRVTITCQASQSIGSNLAWYQQKPGKPPKLLIYKGGTLES GVPSRFSGSGSGTDFTLTISSLQPEDFATYYCQSTAGSSRSGNYGYVFGTGTKVTV LGGGGGSGGGGSGGGGSGGGGSQQLVESGGGLVQPGGSLRLSCAASGFSFTN NYYMCWVRQAPGKGLEWIACMLVGAWGNTYYASWANGRFTISKDNSKNTVYLQ MNSLRAEDSAVYFCARARDYTWTLGGDPETSGFNLWGQGTLVKVSS |
| 19 | **scFv (VL-linker-VH)** 68-18-E11-sc07_(VL_A67G_S68G_T141C; VH_G51C_T101S_T146K) (PRO3744) | DVQMTQSPSSLSASVGDRVTITCQASQSIGSNLAWYQQKPGKPPKLLIYKGGTLES GVPSRFSGSGSGTDFTLTISSLQPEDFATYYCQSTAGSSRSGNYGYVFGCGTKVT VLGGGGGSGGGGSGGGGSGGGGSQQLVESGGGLVQPGGSLRLSCAASGFSFTN NYYMCWVRQAPGKCLEWIACMLVGAWGNTYYASWANGRFTISKDNSKNTVYLQ MNSLRAEDSAVYFCARARDYTWTLGGDPETSGFNLWGQGTLVKVSS |
| *69-06-G04* - *based sequences* | | |
| 20 | **HCDR1** (H27-H42; AHo numbering) | ***GFDLSADYMC*** |
| 21 | **HCDR2** (H57-H76; AHo numbering) | ***CIYIDDDNTYYATWAKG*** |
| 22 | **HCDR3** (H108-H138; AHo numbering) | ***RGYGVYTGAITYFTL*** |
| 23 | **LCDR1** (L24-L42; AHo numbering) | ***QASQSIGTYLA*** |
| 24 | **LCDR2** (L58-L72; AHo numbering) | ***RASTLAS*** |
| 25 | **LCDR2** (L58-L72; AHo numbering) | ***RGGTLAS*** |
| 26 | **LCDR3** (L107-L138; AHo numbering) | ***QSYDSSNT*** |
| 27 | **VH** 69-06-G04-sc05 | QEQLVESGGGLVQPGRSLRLSCAASGFDLSADYMCWVRQAPGKGLEWIACIYIDD DNTYYATWAKGRFTISKDNSKNTVYLQMNSLRAEDTAVYFCARGYGVYTGAITYFT LWGQGTLVLVSS |
| 28 | **VH** 69-06-G04-sc05_(T101S_T146K) | QEQLVESGGGLVQPGRSLRLSCAASGFDLSADYMCWVRQAPGKGLEWIACIYIDD DNTYYATWAKGRFTISKDNSKNTVYLQMNSLRAEDSAVYFCARGYGVYTGAITYFT LWGQGTLVKVSS |
| 29 | **VL** 69-06-G04-sc05 | AFQLTQSPSSLSASVGDRVTITCQASQSIGTYLAWYQQKPGKPPKLLIYRASTLAS GVPSRFSGSGSGTDFTLTISSLQPEDFATYYCQSYDSSNTFGTGTKVTVLG |
| 30 | **VL** 69-06-G04-sc05_(A67G_S68G) | AFQLTQSPSSLSASVGDRVTITCQASQSIGTYLAWYQQKPGKPPKLLIYRGGTLAS GVPSRFSGSGSGTDFTLTISSLQPEDFATYYCQSYDSSNTFGTGTKVTVLG |
| 31 | **VL** 69-06-G04-sc05_(KappaCap) | AFQLTQSPSSLSASVGDRVTITCQASQSIGTYLAWYQQKPGKPPKLLIYRASTLAS GVPSRFSGSGSGTDFTLTISSLQPEDFATYYCQSYDSSNTFGQGTKVEIK |
| 32 | **VL** 69-06-G04-sc05_(A67G_S68G_KappaCap) | AFQLTQSPSSLSASVGDRVTITCQASQSIGTYLAWYQQKPGKPPKLLIYRGGTLAS GVPSRFSGSGSGTDFTLTISSLQPEDFATYYCQSYDSSNTFGQGTKVEIK |
| 33 | **scFv (VL-linker-VH)** 69-06-G04-sc05 | AFQLTQSPSSLSASVGDRVTITCQASQSIGTYLAWYQQKPGKPPKLLIYRASTLAS GVPSRFSGSGSGTDFTLTISSLQPEDFATYYCQSYDSSNTFGTGTKVTVLGGGGG SGGGGSGGGGSGGGGSQEQLVESGGGLVQPGRSLRLSCAASGFDLSADYMCW VRQAPGKGLEWIACIYIDDDNTYYATWAKGRFTISKDNSKNTVYLQMNSLRAEDTA VYFCARGYGVYTGAITYFTLWGQGTLVLVSS |
| 34 | **scFv (VL-linker-VH)** 69-06-G04-sc05_(VL_A67G_S68G) | AFQLTQSPSSLSASVGDRVTITCQASQSIGTYLAWYQQKPGKPPKLLIYRGGTLAS GVPSRFSGSGSGTDFTLTISSLQPEDFATYYCQSYDSSNTFGTGTKVTVLGGGGG SGGGGSGGGGSGGGGSQEQLVESGGGLVQPGRSLRLSCAASGFDLSADYMCW VRQAPGKGLEWIACIYIDDDNTYYATWAKGRFTISKDNSKNTVYLQMNSLRAEDTA VYFCARGYGVYTGAITYFTLWGQGTLVLVSS |
| 35 | **scFv (VL-linker-VH)** 69-06-G04-sc05_(VL_A67G_S68G; VH_T101S_L146K) (PRO3697) | AFQLTQSPSSLSASVGDRVTITCQASQSIGTYLAWYQQKPGKPPKLLIYRGGTLAS GVPSRFSGSGSGTDFTLTISSLQPEDFATYYCQSYDSSNTFGTGTKVTVLGGGGG SGGGGSGGGGSGGGGSQEQLVESGGGLVQPGRSLRLSCAASGFDLSADYMCW VRQAPGKGLEWIACIYIDDDNTYYATWAKGRFTISKDNSKNTVYLQMNSLRAEDSA VYFCARGYGVYTGAITYFTLWGQGTLVKVSS |
| *69-01-E02* - *based sequences* | | |
| 36 | **HCDR1** (H27-H42; AHo numbering) | ***GFSFSSSSYMC*** |
| 37 | **HCDR2** (H57-H76; AHo numbering) | ***CMVTGSSDDTDYASWAKG*** |
| 38 | **HCDR3** (H108-H138; AHo numbering) | ***RAGSAPL*** |
| 39 | **LCDR1** (L24-L42; AHo numbering) | ***QASQSIGLNLA*** |
| 40 | **LCDR2** (L58-L72; AHo numbering) | ***AASYLAS*** |
| 41 | **LCDR2** (L58-L72; AHo numbering) | ***AGGYLAS*** |
| 42 | **LCDR3** (L107-L138; AHo numbering) | ***QSTYDGSSFVFA*** |
| 43 | **VH** 69-01-E02-sc06 | QEQLVESGGGLVQPGGSLRLSCAASGFSFSSSSYMCWVRQAPGKGLEWIACMVT GSSDDTDYASWAKGRFTISKDNSKNTVYLQMNSLRAEDTAVYFCARAGSAPLWG QGTLVLVSS |
| 44 | **VH** 69-01-E02-sc06_(T101S_T146K) | QEQLVESGGGLVQPGGSLRLSCAASGFSFSSSSYMCWVRQAPGKGLEWIACMVT GSSDDTDYASWAKGRFTISKDNSKNTVYLQMNSLRAEDSAVYFCARAGSAPLWG QGTLVKVSS |
| 45 | **VL** 69-01-E02-sc06 | DVQMTQSPSSLSASVGDRVTITCQASQSIGLNLAWYQQKPGKPPKLLIYAASYLAS GVSSRFSGSGSGTDFTLTISSLQPEDFATYYCQSTYDGSSFVFAFGTGTKVTVLG |
| 46 | **VL** 69-01-E02-sc06_(A67G_S68G) | DVQMTQSPSSLSASVGDRVTITCQASQSIGLNLAWYQQKPGKPPKLLIYAGGYLAS GVSSRFSGSGSGTDFTLTISSLQPEDFATYYCQSTYDGSSFVFAFGTGTKVTVLG |
| 47 | **VL** 69-01-E02-sc06_(KappaCap) | DVQMTQSPSSLSASVGDRVTITCQASQSIGLNLAWYQQKPGKPPKLLIYAASYLAS GVSSRFSGSGSGTDFTLTISSLQPEDFATYYCQSTYDGSSFVFAFGQGTKVEIKR |
| 48 | **VL** 69-01-E02-sc06_(A67G_S68G_KappaCap) | DVQMTQSPSSLSASVGDRVTITCQASQSIGLNLAWYQQKPGKPPKLLIYAGGYLAS GVSSRFSGSGSGTDFTLTISSLQPEDFATYYCQSTYDGSSFVFAFGQGTKVEIKR |
| 49 | **scFv** (VL-linker-VH) 69-01-E02-sc06 | DVQMTQSPSSLSASVGDRVTITCQASQSIGLNLAWYQQKPGKPPKLLIYAASYLAS GVSSRFSGSGSGTDFTLTISSLQPEDFATYYCQSTYDGSSFVFAFGTGTKVTVLGG GGGSGGGGSGGGGSGGGGSQEQLVESGGGLVQPGGSLRLSCAASGFSFSSSSY MCWVRQAPGKGLEWIACMVTGSSDDTDYASWAKGRFTISKDNSKNTVYLQMNSL RAEDTAVYFCARAGSAPLWGQGTLVLVSS |
| 50 | **scFv** (VL-linker-VH) 69-01-E02-sc06_(VL_A67G_S68G) | DVQMTQSPSSLSASVGDRVTITCQASQSIGLNLAWYQQKPGKPPKLLIYAGGYLAS GVSSRFSGSGSGTDFTLTISSLQPEDFATYYCQSTYDGSSFVFAFGTGTKVTVLGG GGGSGGGGSGGGGSGGGGSQEQLVESGGGLVQPGGSLRLSCAASGFSFSSSSY MCWVRQAPGKGLEWIACMVTGSSDDTDYASWAKGRFTISKDNSKNTVYLQMNSL RAEDTAVYFCARAGSAPLWGQGTLVLVSS |
| 51 | **scFv** (VL-linker-VH) 69-01-E02-sc06_(VL_A67G_S68G; VH_T101S_L146K) (PRO3696) | DVQMTQSPSSLSASVGDRVTITCQASQSIGLNLAWYQQKPGKPPKLLIYAGGYLAS GVSSRFSGSGSGTDFTLTISSLQPEDFATYYCQSTYDGSSFVFAFGTGTKVTVLGG GGGSGGGGSGGGGSGGGGSQEQLVESGGGLVQPGGSLRLSCAASGFSFSSSSY MCWVRQAPGKGLEWIACMVTGSSDDTDYASWAKGRFTISKDNSKNTVYLQMNSL RAEDSAVYFCARAGSAPLWGQGTLVKVSS |

**Table 2. Other sequences related to the present invention.**

| **SEQ ID** NO: | **Description** | **Sequence** |
|---|---|---|
| 52 | VH3 | EVQLVESGGGLVQPGGSLRLSCAASGFSFSANYYPCWVRQAPGKGLEWIGCIYGGS SDITYDANWTKGRFTISRDNSKNTVYLQMNSLRAEDTAVYYCARSAWYSGWGGDLW GQGTLVTVSS |
| 53 | VH3_(51C) | EVQLVESGGGLVQPGGSLRLSCAASGFSFSANYYPCWVRQAPGKCLEWIGCIYGGS SDITYDANWTKGRFTISRDNSKNTVYLQMNSLRAEDTAVYYCARSAWYSGWGGDLW GQGTLVTVSS |
| 54 | VH1a | QVQLVQSGAEVKKPGSSVKVSCKASGIDFNSNYYMCWVRQAPGQGLEWMGCIYVGS HVNTYYANWAKGRVTITADESTSTAYMELSSLRSEDTAVYYCATSGSSVLYFKFWGQ GTLVTVSS |
| 55 | VH1b | QVQLVQSGAEVKKPGASVKVSCKASGIDFNSNYYMCWVRQAPGQGLEWMGCIYVGS HVNTYYANWAKGRVTMTRDTSISTAYMELSSLRSEDTAVYYCATSGSSVLYFKFWGQ GTLVTVSS |
| 56 | VH4 | QVQLQESGPGLVKPSETLSLTCTVSGIDFNSNYYMCWIRQPPGKGLEWIGCIYVGSHV NTYYANWAKGRVTISVDTSKNQFSLKLSSVTAADTAVYYCATSGSSVLYFKFWGQGT LVTVSS |
| 57 | VH5 | EVQLVQSGAEVKKPGESLKISCKGSGIDFNSNYYMCWVRQMPGKGLEWMGCIYVGS HVNTYYANWAKGQVTISADKSISTAYLQWSSLKASDTAMYYCATSGSSVLYFKFWGQ GTLVTVSS |
| 58 | VH6 | QVQLQQSGPGLVKPSQTLSLTCAISGIDFNSNYYMCWIRQSPGRGLEWLGCIYVGSHV NTYYANWAKGRITINPDTSKNQFSLQLNSVTPEDTAVYYCATSGSSVLYFKFWGQGT LVTVSS |
| 59 | Vkappa1_V1 (L24 is defined here to belong to LFW1) | DIQMTQSPSSLSASVGDRVTITCQASQSINNVLAWYQQKPGKAPKLLIYRASTLASGV PSRFSGSGSGTDFTLTISSLQPEDFATYYCQSSYGNYGDFGQGTKVEIKR |
| 60 | Vkappa1_V1_A-LFW4 (L24 is defined here to belong to LFW1) | DIQMTQSPSSLSASVGDRVTITCQASQSINNVLAWYQQKPGKAPKLLIYRASTLASGV PSRFSGSGSGTDFTLTISSLQPEDFATYYCQSSYGNYGDFGTGTKVTVLG |
| 61 | Vkappa1_V1_A-LFW4_(141C) (L24 is defined here to belong to LFW1) | DIQMTQSPSSLSASVGDRVTITCQASQSINNVLAWYQQKPGKAPKLLIYRASTLASGV PSRFSGSGSGTDFTLTISSLQPEDFATYYCQSSYGNYGDFGCGTKVTVLG |
| 62 | Vλ germline-based FR4 (Sk17) | FGTGTKVTVLG |
| 63 | Vλ germline-based FR4 (Sk12) | FGGGTKLTVLG |
| 64 | Vλ germline-based FR4 | FGGGTQLIILG |
| 65 | Vλ germline-based FR4 | FGEGTELTVLG |
| 66 | Vλ germline-based FR4 | FGSGTKVTVLG |
| 67 | Vλ germline-based FR4 | FGGGTQLTVLG |
| 68 | Vλ germline-based FR4 | FGGGTQLTALG |
| 69 | Vλ germline-based FR4 G141T | FGTGTKLTVLG |
| 70 | Vλ germline-based FR4 G141C | FGCGTKVTVLG |
| 71 | Linker L1 | (GGGGS)ₙ, wherein n=1, 2, 3, 4 or 5 |
| 72 | Linker L1 | GGGGSGGGGS |
| 73 | Linker L2 | (GGGGS)ₙ, wherein n=1, 2, 3, 4, 5, 6, 7 or 8 |
| 74 | Linker L2 | GGGGSGGGGSGGGGSGGGGS |

**Table 3: anti-LILRB2 antibodies of the present invention (modifications relative to original sequences, i.e. reference sequences, if present, are shown in bold and underlined; CDR residues are shown in bold and italic letters.**

| **SEQ ID NO:** | **Description** | **Sequence** |
|---|---|---|
| ***PRO3603*** | | |
| 75 | 68-18-E11-sc02_IgG1_VH (2x) | QQLVESGGGRVQPGGSLRLSCAASGFSFTNNYYMCWVRQAPGKGLEWIACMLVGAWGNTYYAS WANGRFTISKDNSKNTVYLQMNSLRAEDTATYFCARARDYTWTLGGDPETSGFNLWGQGTQVTV SSASTKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPELLGGPSVFLFPPKPK DTLMISRTPEVTCVVVDVSHEDPEVKFNWYVDGVEVHNAKTKPREEQYNSTYRVVSVLTVLHQDWL NGKEYKCKVSNKALPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALHNHYTQKSLSLSPG K |
| 76 | 68-18-E11-sc02_IgG1_VL (2x) | DVQMTQSPSSLSASVGDRVTITCQASQSIGSNLAWYQQKPGKPPKLLIYKASTLESGVPSRFSGSG SGTDFTLTISSLQPEDFATYYCQSTAGSSRSGNYGYVFGQGTKVEIKRTVAAPSVFIFPPSDEQLKS GTASVVCLLNNFYPREAKVQWKVDNALQSGNSQESVTEQDSKDSTYSLSSTLTLSKADYEKHKVYA CEVTHQGLSSPVTKSFNRGEC |
| ***PRO3635*** | | |
| 77 | 68-18-E11-sc02_IgG4_VH (2x) | QQLVESGGGRVQPGGSLRLSCAASGFSFTNNYYMCWVRQAPGKGLEWIACMLVGAWGNTYYAS WANGRFTISKDNSKNTVYLQMNSLRAEDTATYFCARARDYTWTLGGDPETSGFNLWGQGTQVTV SSASTKGPSVFPLAPCSRSTSESTAALGCLVKDYFPEPVTVSWNSGALTSGVHTFPAVLQSSGLYSL SSVVTVPSSSLGTKTYTCNVDHKPSNTKVDKRVESKYGPPCPPCPAPEFLGGPSVFLFPPKPKDTL MISRTPEVTCVVVDVSQEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSLTCLVKGFYPSDIAVEWES NGQPENNYKTTPPVLDSDGSFFLYSRLTVDKSRWQEGNVFSCSVMHEALHNHYTQKSLSLSLGK |
| 78 | 68-18-E11-sc02_IgG4_VL (2x) | DVQMTQSPSSLSASVGDRVTITCQASQSIGSNLAWYQQKPGKPPKLLIYKASTLESGVPSRFSGSG SGTDFTLTISSLQPEDFATYYCQSTAGSSRSGNYGYVFGQGTKVEIKRTVAAPSVFIFPPSDEQLKS GTASVVCLLNNFYPREAKVQWKVDNALQSGNSQESVTEQDSKDSTYSLSSTLTLSKADYEKHKVYA CEVTHQGLSSPVTKSFNRGEC |
| ***PRO4710*** | | |
| 79 | 68-18-E11-sc07_IgG4_VH (2x) | QQLVESGGGLVQPGGSLRLSCAASGFSFTNNYYMCWVRQAPGKGLEWIACMLVGAWGNTYYAS WANGRFTISKDNSKNTVYLQMNSLRAEDTAVYFCARARDYTWTLGGDPETSGFNLWGQGTLVTVS SASTKGPSVFPLAPCSRSTSESTAALGCLVKDYFPEPVTVSWNSGALTSGVHTFPAVLQSSGLYSLS SVVTVPSSSLGTKTYTCNVDHKPSNTKVDKRVESKYGPPCPPCPAPEFLGGPSVFLFPPKPKDTLMI SRTPEVTCVVVDVSQEDPEVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLVKGFYPSDIAVEWESNG QPENNYKTTPPVLDSDGSFFLYSRLTVDKSRWQEGNVFSCSVMHEALHNHYTQKSLSLSLGK |
| 80 | 68-18-E11-sc07_IgG4_VL (2x) | DVQMTQSPSSLSASVGDRVTITCQASQSIGSNLAWYQQKPGKPPKLLIYKGGTLESGVPSRFSGSG SGTDFTLTISSLQPEDFATYYCQSTAGSSRSGNYGYVFGQGTKVEIKRTVAAPSVFIFPPSDEQLKS GTASVVCLLNNFYPREAKVQWKVDNALQSGNSQESVTEQDSKDSTYSLSSTLTLSKADYEKHKVYA CEVTHQGLSSPVTKSFNRGEC |

**Table 4: Examples of anti-PDL1 x CD47 x LILRB2 multispecific antibodies, according to the present invention (modifications relative to original sequences, i.e. reference sequences, if present, are shown in bold and underlined; CDR residues are shown in bold and italic letters).**

| **SEQ ID NO:** | **Description** | **Sequence** |
|---|---|---|
| **PRO4672 (37-20-B03-based PDL1-BD)** | | |
| 81 | (scFv)2-Fc-(scFv)2, hole | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGKGGGGSGGGGSDVQMTQSPSSLSASVGDRVTITCQASQSIGSNLAWYQQ KPGKPPKLLIYKGGTLESGVPSRFSGSGSGTDFTLTISSLQPEDFATYYCQSTAGSSRSGNYGYVFG CGTKVTVLGGGGGSGGGGSGGGGSGGGGSQQLVESGGGLVQPGGSLRLSCAASGFSFTNNYYM CWVRQAPGKCLEWIACMLVGAWGNTYYASWANGRFTISKDNSKNTVYLQMNSLRAEDSAVYFCA RARDYTWTLGGDPETSGFNLWGQGTLVKVSS |
| | 70-26-B04-sc03_hole-Fc_(G4S)2_68-18-E11-sc07 | |
| 82 | (scFv)2-Fc-(scFv)2, knob 37-20-B03-sc18_knob-Fc_(G4S)2_68-18-E11-sc07 | DIQMTQSPSSLSASVGDRVTITCQASQSIGTYLAWYQQKPGKPPKLLIYRGGILASGVPSRFSGSGS GTDFTLTISSLQPEDFATYYCQSNFYSDSTTIGPNAFGTGTKVTVLGGGGGSGGGGSGGGGSGGG |
| | | GSQQLVESGGGLVQPGGSLRLSCAASGFSFNSDYWIYWVRQAPGKGLEWIASIYGGSSGNTQYAS WAQGRFTISKDNSKNTVYLQMNSLRAEDSAVYFCARGYVDYGGATDLWGQGTLVKVSSEPKSSD KTHTCPPCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVCTLPPS REEMTKNQVSLWCLVKGFYPSDIAVEWESNGQPENNYKTTPPVLDSDGSFFLYSKLTVDKSRWQQ GNVFSCSVMHEALHNHYTQKSLSLSPGKGGGGSGGGGSDVQMTQSPSSLSASVGDRVTITCQAS QSIGSNLAWYQQKPGKPPKLLIYKGGTLESGVPSRFSGSGSGTDFTLTISSLQPEDFATYYCQSTAG SSRSGNYGYVFGCGTKVTVLGGGGGSGGGGSGGGGSGGGGSQQLVESGGGLVQPGGSLRLSC AASGFSFTNNYYMCWVRQAPGKCLEWIACMLVGAWGNTYYASWANGRFTISKDNSKNTVYLQMN SLRAEDSAVYFCARARDYTWTLGGDPETSGFNLWGQGTLVKVSS |
| **PRO4687 (37-20-B03-based PDL1-BD)** | | |
| 83 | 70-26-B04-sc03 _ hole-Fc_(G4S)2_68-18-E11-sc07 | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGKGGGGSGGGGSDVQMTQSPSSLSASVGDRVTITCQASQSIGSNLAWYQQ KPGKPPKLLIYKGGTLESGVPSRFSGSGSGTDFTLTISSLQPEDFATYYCQSTAGSSRSGNYGYVFG CGTKVTVLGGGGGSGGGGSGGGGSGGGGSQQLVESGGGLVQPGGSLRLSCAASGFSFTNNYYM CWVRQAPGKCLEWIACMLVGAWGNTYYASWANGRFTISKDNSKNTVYLQMNSLRAEDSAVYFCA RARDYTWTLGGDPETSGFNLWGQGTLVKVSS |
| 84 | 37-20-B03-sc18 (noSK)_lgG_K_(G4S)2_ 68-18-E11-sc07_HC | QQLVESGGGLVQPGGSLRLSCAASGFSFNSDYWIYWVRQAPGKGLEWIASIYGGSSGNTQYASW AQGRFTISKDNSKNTVYLQMNSLRAEDTAVYFCARGYVDYGGATDLWGQGTLVTVSSASTKGPSV FPLAPSSKSTSGGTAALGCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNGKEYKCKVS NKALPAPIEKTISKAKGQPREPQVCTLPPSREEMTKNQVSLWCLVKGFYPSDIAVEWESNGQPENN YKTTPPVLDSDGSFFLYSKLTVDKSRWQQGNVFSCSVMHEALHNHYTQKSLSLSPGKGGGGGSGG GGSDVQMTQSPSSLSASVGDRVTITCQASQSIGSNLAWYQQKPGKPPKLLIYKGGTLESGVPSRFS GSGSGTDFTLTISSLQPEDFATYYCQSTAGSSRSGNYGYVFGCGTKVTVLGGGGGSGGGGSGGG GSGGGGSQQLVESGGGLVQPGGSLRLSCAASGFSFTNNYYMCWVRQAPGKCLEWIACMLVGAW |
| | | GNTYYASWANGRFTISKDNSKNTVYLQMNSLRAEDSAVYFCARARDYTWTLGGDPETSGFNLWG QGTLVKVSS |
| 85 | 37-20-B03-sc18(kappacap)_Ck_VL | DIQMTQSPSSLSASVGDRVTITCQASQSIGTYLAWYQQKPGKPPKLLIYRGGILASGVPSRFSGSGS GTDFTLTISSLQPEDFATYYCQSNFYSDSTTIGPNAFGQGTKVEIKRTVAAPSVFIFPPSDEQLKSGT ASVVCLLNNFYPREAKVQWKVDNALQSGNSQESVTEQDSKDSTYSLSSTLTLSKADYEKHKVYACE VTHQGLSSPVTKSFNRGEC |
| **PRO5052** (PRO4672 with 33-03-G02-based PDL1-BD) | | |
| 86 | (scFv)2-Fc-(scFv)2, hole 70-26-B04-sc03_hole-Fc_(G4S)2_68-18-E11-sc07 | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGKGGGGSGGGGSDVQMTQSPSSLSASVGDRVTITCQASQSIGSNLAWYQQ KPGKPPKLLIYKGGTLESGVPSRFSGSGSGTDFTLTISSLQPEDFATYYCQSTAGSSRSGNYGYVFG CGTKVTVLGGGGGSGGGGSGGGGSGGGGSQQLVESGGGLVQPGGSLRLSCAASGFSFTNNYYM CWVRQAPGKCLEWIACMLVGAWGNTYYASWANGRFTISKDNSKNTVYLQMNSLRAEDSAVYFCA RARDYTWTLGGDPETSGFNLWGQGTLVKVSS |
| 87 | (scFv)2-Fc-(scFv)2, knob 33-03-G02-sc24_knob-Fc_(G4S)2_68-18-E11-sc07 | DIQMTQSPSSLSASVGDRVTITCQASQSINDYLAWYQQKPGKAPKLLIYKGGTLASGVPSRFSGSG SGTDFTLTISSLQPEDFATYYCQQGYIITDIDNVFGTGTKVTVLGGGGGSGGGGSGGGGSGGGGSQ VQLQESGPGLVKPSETLSLTCKASGFSFSSGYDMCWVRQPPGKGLEWIACVVAGSVDITYYASWA KGRVTISKDSSKNQVSLKLSSVTAADSAVYYCARKDAYSDAFNLWGQGTLVKVSSEPKSSDKTHTC PPCPAPELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVCTLPPSREEMT KNQVSLWCLVKGFYPSDIAVEWESNGQPENNYKTTPPVLDSDGSFFLYSKLTVDKSRWQQGNVFS CSVMHEALHNHYTQKSLSLSPGKGGGGSGGGGSDVQMTQSPSSLSASVGDRVTITCQASQSIGSN LAWYQQKPGKPPKLLIYKGGTLESGVPSRFSGSGSGTDFTLTISSLQPEDFATYYCQSTAGSSRSG NYGYVFGCGTKVTVLGGGGGSGGGGSGGGGSGGGGSQQLVESGGGLVQPGGSLRLSCAASGFS FTNNYYMCWVRQAPGKCLEWIACMLVGAWGNTYYASWANGRFTISKDNSKNTVYLQMNSLRAED SAVYFCARARDYTWTLGGDPETSGFNLWGQGTLVKVSS |
| PRO5055 (PRO4687 with 33-03-G02-based POL1-BD) | | |
| 88 | 70-26-B04-sc03_hole-Fc_(G4S)2_68-18-E11-sc07 | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGKGGGGSGGGGSDVQMTQSPSSLSASVGDRVTITCQASQSIGSNLAWYQQ KPGKPPKLLIYKGGTLESGVPSRFSGSGSGTDFTLTISSLQPEDFATYYCQSTAGSSRSGNYGYVFG CGTKVTVLGGGGGSGGGGSGGGGSGGGGSQQLVESGGGLVQPGGSLRLSCAASGFSFTNNYYM CWVRQAPGKCLEWIACMLVGAWGNTYYASWANGRFTISKDNSKNTVYLQMNSLRAEDSAVYFCA RARDYTWTLGGDPETSGFNLWGQGTLVKVSS |
| 89 | 33-03-G02-sc24 (noSK)_IgG_K_(G4S)2_ 68-18-E11-sc07_HC | QVQLQESGPGLVKPSETLSLTCKASGFSFSSGYDMCWVRQPPGKGLEWIACVVAGSVDITYYASW AKGRVTISKDSSKNQVSLKLSSVTAADTAVYYCARKDAYSDAFNLWGQGTLVTVSSASTKGPSVFP LAPSSKSTSGGTAALGCLVKDYFPEPVTVSWNSGALTSGVHTFPAVLQSSGLYSLSSVVTVPSSSLG TQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSNK ALPAPIEKTISKAKGQPREPQVCTLPPSREEMTKNQVSLWCLVKGFYPSDIAVEWESNGQPENNYK TTPPVLDSDGSFFLYSKLTVDKSRWQQGNVFSCSVMHEALHNHYTQKSLSLSPGKGGGGGSGGG GSDVQMTQSPSSLSASVGDRVTITCQASQSIGSNLAWYQQKPGKPPKLLIYKGGTLESGVPSRFSG SGSGTDFTLTISSLQPEDFATYYCQSTAGSSRSGNYGYVFGCGTKVTVLGGGGGSGGGGSGGGG SGGGGSQQLVESGGGLVQPGGSLRLSCAASGFSFTNNYYMCWVRQAPGKCLEWIACMLVGAWG NTYYASWANGRFTISKDNSKNTVYLQMNSLRAEDSAVYFCARARDYTWTLGGDPETSGFNLWGQ GTLVKVSS |
| 90 | 33-03-G02-sc24(kappa-cap)_Ck_VL | DIQMTQSPSSLSASVGDRVTITCQASQSINDYLAWYQQKPGKAPKLLIYKGGTLASGVPSRFSGSG SGTDFTLTISSLQPEDFATYYCQQGYIITDIDNVFGQGTKVEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGEC |

Throughout the text of this application, should there be a discrepancy between the text of the specification (*e. g*., Tables 1 to 4) and the sequence listing, the text of the specification shall prevail.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all subcombinations of the various embodiments and elements thereof are also specifically embraced by the present invention and are disclosed herein just as if each and every such sub-combination was individually and explicitly disclosed herein.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

To the extent possible under the respective patent law, all patents, applications, publications, test methods, literature, and other materials cited herein are hereby incorporated by reference.

The following Examples illustrates the invention described above, but is not, however, intended to limit the scope of the invention in any way. Other test models known as such to the person skilled in the pertinent art can also determine the beneficial effects of the claimed invention.

### Examples

### Example 1: Generation and testing of antibodies directed against human LILRB2:

### Aim of the project

The goal of the project was to generate humanized monoclonal antibody variable domains that specifically bind to and neutralize the biological effect of human LILRB2. A further goal was to identify anti-LILRB2 antibody variable domains that are potent and stable enough for incorporation into multispecific antibody formats.

### 1.1. Immunization

Three different immunization protocols were applied to a total of 12 rabbits to obtain an optimal immune response against human LILRB2. Rabbits were immunized with recombinantly produced and purified LILRB2 extracellular domain (ECD) (Sino Biological, Cat. No. 14132-H08H). Prior to immunization, the quality of recombinant human LILRB2 was analyzed by the manufacturer for purity by SDS-page. The first group of three rabbits (protocol 1) received 4 injections of 200 µg each throughout 33 days. The second group of six rabbits (protocol 2) received 4 injections of 200 µg each throughout 70 days. The third group of three rabbits (protocol 3) received 5 injections of 200 µg each through a period of 108 days. During the course of the immunization, the strength of the humoral immune response against the antigen was qualitatively assessed by determining the maximal dilution (titer) for the serum of each rabbit that still results in detectable binding of the polyclonal serum antibodies to the antigen. Serum antibody titers against the immobilized antigen (recombinant human LILRB2) were assessed using an enzyme-linked immunosorbent assay (ELISA). All nine rabbits immunized with purified human LILRB2 showed high titers with EC₅ up to 1:1.5 × 10⁷.

### 1.2. Sorting

Prior to the hit identification procedure, a flow-cytometry-based sorting procedure was performed in the presence of R-Phycoerythrin (RPE)-labelled human or cynomolgus monkey LILRB2 ECD, which allows the specific detection and isolation of high-affinity LILRB2-binding B-cells. A total of 9.05 × 10⁷ lymphocytes derived from nine rabbits were analyzed in this sorting campaign. Of these, a total of 10,560 B-cells expressing LILRB2-specific antibodies (IgG) were isolated and individually cultured as single clones for three to four weeks.

### 1.3. Hit identification

### General remarks

For hit identification, direct ELISA screens were performed to assess binding to human LILRB2. Binding to cynomolgus and rhesus monkey LILRB2 was evaluated by CELISA using transiently transfected cells expressing cynomolgus or rhesus LILRB2.

### Binding to human LILRB2 by ELISA

For hit identification, *i. e*. the identification of B cell clones that produce antibodies binding to LILRB2, cell culture supernatants of the above 10,560 B cell clones were screened for the presence of antibodies that bind to human LILRB2 ECD by ELISA. This primary hit identification procedure was performed with non-purified antibodies from the cell culture supernatants, as the scale of the high-throughput culture does not allow for purification of the individual rabbit antibodies. The use of cell supernatants allows for ranking of large numbers of antibodies. The ELISA method used assesses the "quantity" of rabbit IgGs bound to recombinant human LILRB2, however it does not provide any information regarding the affinity or the concentration of the antibodies. In this assay, supernatants from 1,423 B cell clones produced a signal that was above background.

### Inhibition of HLA-G and HLA-A02 binding to human LILRB2 expressing cells

All 1,423 human LILRB2-binding supernatants were further analyzed for their potential to inhibit the binding of HLA-G tetramers to LILRB2-expressing cells. For some of the supernatants also the potential to inhibit binding of HLA-A02 tetramers to LILRB2-expressing cells was analyzed.

For this purpose, a flow-cytometry based assay was developed. Inhibition of binding of PE-labelled HLA-G tetramers or HLA-A02 multimers, respectively, to CHO cells stably transfected with human LILRB2 by B-cell supernatants was assessed by flow-cytometry. Since the assays could be performed with B cell supernatant as matrix and were sensitive and precise enough, both assays were used for screening. The blocking activity of each B cell supernatant was tested using single well analysis (no dose-responses were performed). Therefore, the extent of inhibition observed is not only dependent on the IgG properties, but also on the concentration of rabbit IgGs in the B cell supernatant.

661 out of 1,423 clones blocked the interaction of human LILRB2 and human HLA-G tetramer by more than 30 %. 94 out of 931 clones blocked the interaction of human LILRB2 and HLA-A02 by more than 50 %.

### Species cross-reactivity (binding to cynomolgus monkey and rhesus monkey LILRB2 by CELISA)

All 1,423 hits identified in the primary screening were analyzed for species cross-reactivity to cynomolgus monkey and rhesus monkey LILRB2 by a cell-based ELISA (CELISA). The ability of the antibodies to cross-react with cyno and rhesus LILRB2 was assessed by CELISA using transiently transfected HEK293T cells expressing rhesus or cynomolgus LILRB2 with an N-terminal V5-tag.

141 (9.9 %) and 51 clones (3.6 %) showed binding to cynomolgus monkey and rhesus monkey LILRB2, respectively. Overall, there were only a few antibodies identified showing cross-reactivity to either of the two monkey species.

### Specificity assessment and mapping of binding region (binding to human LILRA1 and to human LILRB2/LILRA1 chimeras by CELISA)

All 1,423 hits identified in the primary screening were analyzed for cross-reactivity to LILRA1, the LILR family member with the highest sequence identity to LILRB2, by a cell-based ELISA (CELISA). The ability of the antibodies to selectively bind to LILRB2 versus LILRA1 was performed using full-length LILRA1 transiently transfected HEK293T cells. 1,177 clones show binding with an optical density (OD) below 1 and are therefore potentially not binding to LILRA1.

LILRB2 (UniProt: Q8N423) is a single-pass transmembrane protein possessing an extracellular domain of four Ig-like domain and an intracellular domain with 3 ITIM-containing motifs. To map the binding regions of the anti-LILRB2 antibodies, chimeric cDNA constructs were generated where each of the extracellular Ig-like domains of LILRB2 was replaced individually by the corresponding Ig-like domain of LILRA1. The clones potentially not showing binding to LILRA1 were tested for binding to cells transiently transfected with the 4 different LILRB2/LILRA1 chimeras. Domain specificity was used to group the LILRB2 specific antibodies into bins.

86 antibodies showed binding to the N-terminal Ig-like domain 1 of LILRB2, 80 to Ig-like domain 2, 2 to Ig-like domain 3 and 323 to Ig-like domain 4. For the rest of the antibodies no binding region was identified.

### Determination of binding affinity to human LILRB2

In a secondary hit identification procedure, information on the binding affinities to human LILRB2 of the approximately 1,177 monoclonal rabbit antibodies that were qualified as positive during the primary screening and were not binding to LILRA1 were determined by surface plasmon resonance (SPR).

For this affinity screening by SPR, an antibody specific for the Fc region of rabbit IgGs was immobilized on a sensor chip (SPR-24 Pro Affinity Sensor, High Capacity Amine, Bruker) using a standard amine-coupling procedure. Rabbit monoclonal antibodies in B cell supernatants were captured by the immobilized anti-rabbit IgG antibody. After capturing of the monoclonal antibodies, human LILRB2 ECD (extracellular domain) was injected into the flow cells for 3 min at a concentration of 45 nM, and dissociation of the protein from the IgG captured on the sensor chip was allowed to proceed for 5 min. The apparent dissociation (k_{d}) and association (kₐ) rate constants and the apparent dissociation equilibrium constant (K_{D}) were calculated with the analysis software (Analyzer, Bruker) using the 1:1 Langmuir binding model.

For 817 anti-LILRB2 antibodies binding affinities to human LILRB2 could be measured. They showed dissociation constants (K_{D}) ranging from 4.8 × 10⁻¹³ M to 1.2 × 10⁻⁷ M. 50 % of the antibodies analyzed had a K_{D} below 0.5 nM.

### 1.4 Humanization and scFv production

The CDRs of the identified rabbit clones were grafted onto Numab's proprietary VH/VL frameworks to produce stable scFvs, as described in the patent applications WO 2022/136675. Different grafting variants were designed and produced.

### 1.5 Manufacture of suitable humanized anti-LILRB2 binding domains for characterization (scFv format)

The expression of the scFv was performed in CHO cells using the ExpiCHO Expression System (Thermo Fisher Scientific). Expression was conducted according to manufacturer's instructions. Proteins were purified from clarified harvest by affinity chromatography (Protein L and/or Protein A). If necessary, variant scFvs were polished by SE-chromatography to a final monomeric content > 95 %. For quality control of the manufactured material, standard analytical methods such as ESI-MS, SE-HPLC, UV₂₈₀ and SDS-PAGE were applied.

The mass of the scFvs has for example been verified by the following ESI-MS standard method. Manufactured scFvs were 5 fold diluted with 1 % TFA. Two µl of sample were injected into an ACQUITY UPLC@ BioResolve-RP-mAb 2.7 µm 2.1×150 mm, 450 Å column (Waters, USA) and desalted using a gradient from 15 % to 85 % buffer B (0.1 % formic acid, 75 % propan-2-ol in acetonitrile) at a flow rate of 200 µl/min at 50°C. The MS analysis was performed on a Synapt G2 mass spectrometer directly coupled to the UPLC station. Mass spectra were acquired in the positive-ion mode by scanning the m/z range from 400 to 5,000 Da with a scan duration of 1 s and an interscan delay of 0.1 s. The data were recorded with the MassLynx 4.2 Software (both Waters, UK). Where possible, the recorded m/z data of single peaks were deconvoluted into mass spectra by applying the maximum entropy algorithm MaxEnt1 (MaxLynx).

The monomeric content of the manufactured scFvs has been determined for example by using the following standard SE-HPLC method. Five µg of the respective scFv, typically present at a concentration of from 0.1 to 10 mg/ml , were injected onto a Shodex KW402.5-4F column using a Hitachi Chromaster HPLC system at 25°C and a flow rate of 0.35 ml/min. The mobile phase was 50 mM sodium acetate, 250 mM sodium chloride in water at pH 6.0. Elution profile was monitored at 280 nm.

Production details of the LILRB2 scFvs are summarized in Table 5.

### 1.6 Manufacture of anti-LILRB2 antibodies (IgG format)

For the 68-18-E11 clone, some IgG variants have been prepared. The manufacture of these anti-LILRB2 antibodies was performed as follows.

Expression was performed in ExpiCHO-S cells using the transient ExpiCHO expression system (Gibco). Expression cultures were cultivated in batch using shaking flasks for 8 days at 37°C. The culture supernatants were separated from cells by centrifugation followed by a 0.22 µm sterile filtration.

Target proteins were captured from the clarified culture supernatants by protein A affinity chromatography and polished by a size-exclusion chromatography (SEC) step, except for the reference molecule PRO3851 (Innovent), which was polished by a cation-exchange chromatography (CIEX) step. For some molecules, no post-capture polishing step was necessary. For quality control of the manufactured material, standard analytical methods such as SE-HPLC, SDS-PAGE, and UV₂₈₀ were used.

For PRO3603, no SEC-polishing step was necessary due to high post-capture monomeric purity. For PRO3603, a simple dialysis against the respective final buffer in a dialysis membrane was performed instead. PRO3635 and PRO4710 on the other hand were polished by SEC, as described below. The manufacturing details of produced anti-LILRB2 antibodies are also summarized in Table 5.

**Table 5: Manufacture of anti-LILRB2 scFvs and IgGs**

| **Protein ID** | **Construct ID** | **Final titer [mg/L]** | **Final purity by SE-HPLC [% monomer]** |
|---|---|---|---|
| PRO3744 | 68-18-E11-sc07 | 166.8 | 99.5 |
| PRO3696 | 69-01-E02-sc06 | 655.8 | 99.4 |
| PRO3697 | 69-06-G04-sc05 | 365.6 | 99.2 |
| PRO3603 (IgG1) | 68-18-E11-sc02 | 108 | 99.7 (after capture) |
| PRO3635 (IgG4) | 68-18-E11-sc02 | 116 | 99.7 |
| PRO4710 (IgG4) | 68-18-E11-sc07 | 165 | 97.8 |
| PRO2518 (Reference) | J19.H1 (Jounce) | 130 | 98.9 (after capture) |
| PRO2734 (Reference) | Merck | 146 | 96.4 (after capture) |
| PRO3483 (Reference) | NGM | 77 | 94.7 |

### 1.7 Pharmacodynamic characterization of suitable humanized anti-LILRB2 binding domains (scFv format)

### Binding affinity to LILRB2

Affinities of the selected anti-LILRB2 scFvs to human and cynomolgus LILRB2 were determined by SPR analysis using an SPR 24 system (Sierra Sensor - Bruker). Human or cynomolgus LILRB2 were immobilized on a CMD200M SPR sensor prism chip (Xantec). Then LILRB2 scFvs were injected at concentrations of 10 nM, 2 nM and 0.4 nM on the sensor chip over spots with immobilized human LILRB2. Empty spots were used as references. After each analyte cycle, the surface was regenerated with 3 M MgCl₂ solution, thereby allowing the next analyte cycle to have only free ligand available. Binding affinity K_{D} was globally calculated by the Bruker SPR software based on the binding association (*k*ₒₙ) and dissociation (*k*_{off}) rate, by fitting a 1:1 Langmuir model to the curves obtained by the cycles with the three different concentrations of the analyte. The binding affinities towards human LILRB2 are summarized in Table 6. None of the tested anti-LILRB2 scFvs showed measurable binding to cynomolgus LILRB2.

**Table 6: Affinity of anti-LILRB2 scFvs to human LILRB2.**

| **Protein ID** | **scFv** | **Affinity to human LILRB2** | | |
|---|---|---|---|---|
| | Clone ID | kₐ (M⁻¹ s⁻¹) | k_{d} (s⁻¹) | K_{D}(M) |
| PRO3744 | 44-03-A03-sc01 | 7.71E+05 | 1.14E-04 | 1.48E-10 |
| PRO3696 | 69-01-E02-sc06 | 7.36E+05 | 1.66E-04 | 2.26E-10 |
| PRO3697 | 69-06-G04-sc05 | 9.74E+05 | 1.28E-04 | 1.31E-10 |

### Binding of anti-LILRB2 scFvs to CHO K1 cells expressing human LILRB2

To test binding of Numab's anti-LILRB2 scFvs PRO3696 and PRO3744 to CHO K1 cells constitutively expressing human LILRB2, a flow cytometry-based assay was developed.

For cell binding experiments, CHO K1 cells expressing human LILRB2 were seeded in a 96-well plate (50,000 cells per well) and 3-fold serial dilutions of the molecules to test were added at concentrations starting at 100 nM to the plates and incubated for 60 min at 4°C on a shaker. Supernatants were removed and detection reagent (Numab's anti-framework antibody conjugated to Alexa Fluor 647, in-house) was added for flow cytometry analysis. The plates were measured with the flow cytometer Novocyte with a flow rate of 120 µl/min and the geometric mean of the fluorescence intensity of single cells was calculated. Obtained MFI values were plotted as function of log-transformed concentrations and ECso values were calculated by using GraphPad prism software.

Table 7 summarises the cell-binding potency data, presenting the ECso and maximum binding values for PRO3696 and PRO3744. The data revealed robust binding of the scFvs PRO3744 and PRO3696 to CHO cells that express human LILRB2, with ECso values in the range of 1 nM.

**Table 7: Binding of anti-LILRB2 scFvs to CHO K1 cells expressing human LILRB2**

| **PRO ID** | **EC₅₀ [nM]** | **Maximum binding (max. MFI sample)** |
|---|---|---|
| PRO3744 | 0.881 | 554'603 |
| PRO3696 | 0.796 | 760'006 |

### Binding of anti-LILRB2 scFvs to different human and cynomolgus PBMC cell types

Binding of the anti-LILRB2 scFv PRO3744 to different PBMC cell types from human as well as from cynomolgus monkeys was examined within the specified subset of T cells, B cells, and myeloid cells. In particular, the ability of anti-LILRB2 scFv PRO3744 to target HLA-DR+ CD14+ monocytes and HLA-DR+ CD14- APCs from human and cynomolgus monkey was investigated. The method used to assess the binding to these cell-types was analogous to the method described in the preceding section.

The results of the cell-binding measurements are summarized in Table 8. Besides binding to human HLA-DR+ CD14+ monocytes and human HLA-DR+ CD14- APCs, no binding to any other human PBMC cell types was observed. Also, no significant binding to cynomolgus monkey PBMC cell types was observed.

### Neutralization of LILRB2 ligand interaction (HLA-G, HLA-A3)

To test the potency of the anti-LILRB2 scFvs to block the binding of known LILRB2 ligands to LILRB2, the binding of HLA-G (complex with human H2AFX, RIIPRHLQL, APC labeled, from Creative Biolabs) and HLA-A3 (HLA-A0301 dextramer, PE labelled, from ImmuDex) to CHO cells expressing human LILRB2 was tested in the presence of the anti LILRB2 scFvs PRO3744, PRO3696 and PRO3697 using flow cytometry.

LILRB2 expressing CHO cells (stably transduced to express human LILRB2 sequence, Q8N423) were seeded in a 96-well plate (50,000 cells per well). 4-fold serial dilutions of the molecules to test including benchmark anti-LILRB2 antibody J-19 analogue (positive control) were added at concentrations ranging from 160 nM to 0.2 pM to the plates and incubated for 60 min at 4°C on a shaker. Supernatants were removed and APC-labelled HLA-G tetramer (from CreativeBiolabs) or HLA-A0301 dextramer labeled with PE (from ImmuDex) were added to the plates at a final concentration of 100 pM (HLA-A0301 dextramer) or at a final dilution of 1:5000 (HLA-G tetramer). After 30 min incubation at 4°C, cells were washed and 50 µl/well of pre-diluted Cytofix solution (1:10 in ddH₂0, from BD Bioscience) were added for flow cytometry analysis. The plates were measured with the flow cytometer Novocyte with a flow rate of 120 µl/min and the geometric mean of the PE and APC fluorescence intensity of bound LILRB2 ligand, HLA-G or HLA-A3, to single cells was calculated. Obtained MFI values were plotted as function of log-transformed concentrations and ECso values were calculated by using GraphPad prism software.

The potency data for inhibition of LILRB2/HLA-G and LILRB2/HLA-A3 are summarized in Table 9. The IC₅₀ as well as maximum inhibition values for the anti-LILRB2 scFvs PRO3744, PRO3696 and PRO3697 were normalized to the control IgG antibody J-19 (PRO2518), which exhibits high affinity binding to human LILRB2.

Although being monovalent, all tested scFvs, namely PRO3744, PRO3696, and PRO3697, demonstrated potent and comprehensive inhibition of the LILRB2/HLA-G interaction with potencies similar to the bivalent IgG antibody J-19 (PRO2518). Notably, PRO3744 also achieved complete neutralization of the HLA-A3/LILRB2 interaction, while PRO3696 exhibited partial inhibition, and PRO3697 did not impede HLA-A3 binding.

**Table 8: Binding to human and cyno PBMCs**

| | | **Human (2 donors)** | | | | **Cynomolgus monkey (2 donors)** | | | |
|---|---|---|---|---|---|---|---|---|---|
| **PRO ID** | **ConcenTration [nM]** | **HLA-DR+ CD14+ [geoMFI × 1000]** | **HLA-DR+ CD14-[geoMFI × 1000]** | **B cells (CD20+) [geoMFI × 1000]** | **T cells (CD3+) [geoMFI × 1000]** | **HLA-DR+ CD14+ [geoMFI × 1000]** | **HLA-DR+ CD14-[geoMFI × 1000]** | **B cells (CD20+) [geoMFI × 1000]** | **T cells (CD3+) [geoMFI × 1000]** |
| PRO3744 | 1.6 | 16.0 ± 5.1 | 3.5 ± 2.7 | No binding | No binding | 0.38 ± 0.04 | No binding | No binding | No binding |
| | 200 | 21.1 ± 5.8 | 3.6 ± 2.9 | No binding | No binding | 1.12 ± 0.50 | No binding | No binding | No binding |
| PRO3744 x-linked *) | 1.6 | 27.1 ± 10.9 | 5.4 ± 4.5 | No binding | No binding | 0.43 ± 0.16 | No binding | No binding | No binding |
| | 200 | 37.2 ± 12.4 | 7.5 ± 6.3 | No binding | No binding | 7.68 ± 2.79 | No binding | No binding | No binding |

| | | | | | | | | | |
|---|---|---|---|---|---|---|---|---|---|
| *) X-linked: cross linked with an anti-framework antibody | | | | | | | | | |

**Table 9: LILRB2 neutralization potency**

| | **LILRB2 / HLA-G** | | | **LILRB2 / HLA-A3** | | |
|---|---|---|---|---|---|---|
| **PRO ID** | **IC₅₀ [nM]** | **Rel. IC₅₀ (IC₅₀ J-19 / IC₅₀ sample)** | **Rel. max. inhibition % (rel. to J-19)** | **IC₅₀ [nM]** | **Rel. IC₅₀ (IC₅₀ J-19 / IC₅₀ sample)** | **Rel. max. inhibition % (rel. to J-19)** |
| PRO3744 | 0.973 | 0.684 | 99.1 | 0.893 | 0.683 | 96.7 |
| PRO3696 | 1.085 | 0.635 | 98.4 | 0.867 | 0.679 | 76.2 |
| PRO3697 | 0.593 | 1.160 | 99.5 | no inhibition | NA | 9.5 |

### LILRB2 selectivity assessment

To assess whether the anti-LILRB2 antibodies selectively bind to LILRB2 and not to other members of protein family LILRA and LILRB, a cellular ELISA (CELISA) using transiently transfected CHO cells was developed.

The selectivity assessment was performed as follows. The day before CELISA, CHO cells seeded in a 96-well cell culture plate were transiently transfected with plasmids encoding LILRA1 (identifier: 075019-1), LILRA2 (Q8N149-1), LILRA3 (Q8N6C8-1), LILRA4 (P59901-1), LILRA5 (A6NI73-1), LILRA6 (Q6PI73-1), LILRB1 (Q8NHL6-1), LILRB2 (Q8N423-2), LILRB3 (075022-1), LILRB4 (Q8NHJ6-1) or LILRB5 (075023-1). Next day, dilution series of molecules to test ranging from 100 nM to 6.4 pM were added to the plates of transfected CHO cells and incubated for 1 h at 37°C in the incubator. As a positive control to confirm successful expression of proteins of interest, transfected CHO cells were also stained with an antibody directed against V5 tag (R&D Systems, cat. MAB8926), as all LILRA and LILRB constructs were designed to express V5 tag at the N-terminus of the proteins of interest. Supernatants were removed and detection reagent (rabbit anti-human Fc fragment, HRP-conjugated, from Jackson ImmunoResearch, cat. 309-035-008) was added to the wells. Plates were incubated for 45 min at 37°C in the incubator. Next, cells were washed and 50 µl of TMB substrate was added to each well. HRP reaction was stopped until sufficient color development was achieved by adding 50 µl of stopping solution (1M HCl) to each well. Plates were read by optical density (OD) measurement at 450 nm and at 690 nm as reference wavelength using BioTek Synergy Neo2 multimode reader. Obtained OD values were plotted as function of log-transformed concentrations and ECso values were calculated by using GraphPad prism software.

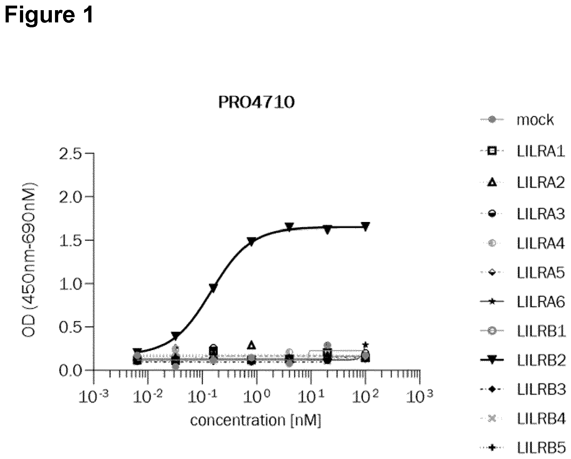

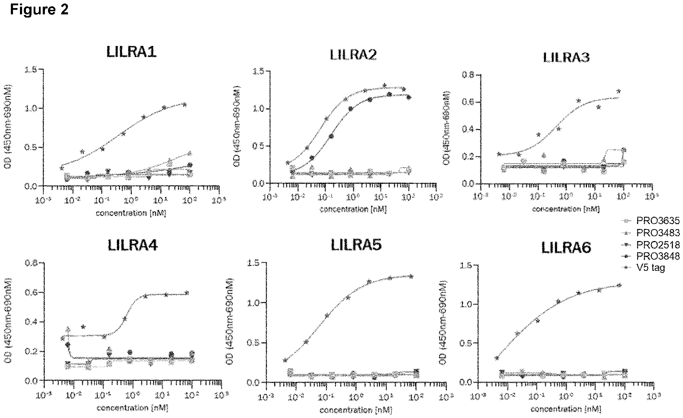

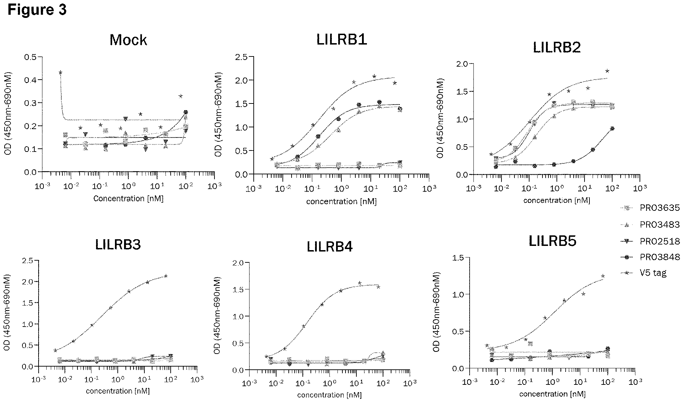

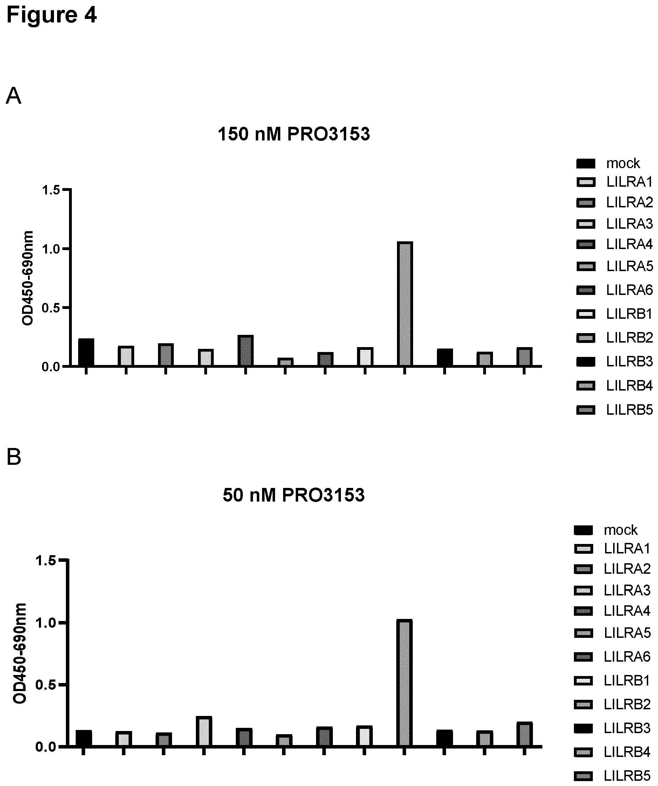

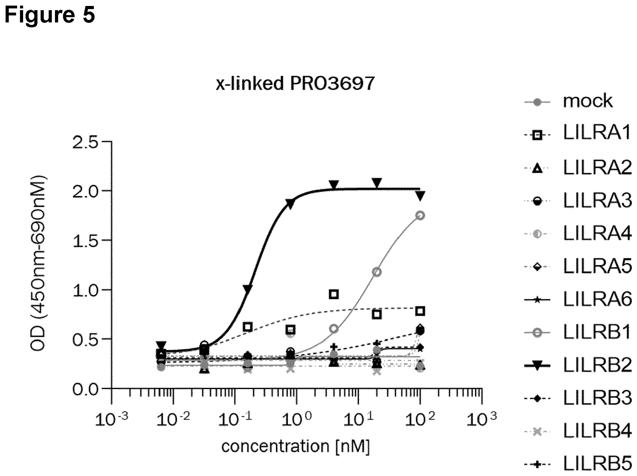

As shown in Figures 1, 2 and 3, PRO4710, the IgG4 variant of scFv PRO3744 (68-18-E11-sc07) as well as PRO3635, the (IgG variant of scFv PRO3064 (68-18-E11-sc02), showed selective binding to LILRB2 while exhibiting no binding to any one of the tested LILRA family members or to LILRB1, LILRB3, LILRB4 or LILRB5. The scFv PRO3153 (69-01-E02-sc03), *i. e*. a precursor of PRO3696 (69-01-E02-sc06), exhibited the same selectivity for LILRB2 as PRO4710, when tested at a concentration of 150 nM (see Figure 4). On the other hand, cross-linked PRO3697 (69-06-G04-sc05) showed, besides strong binding to LILRB2, weak binding to LILRB1, but no binding to any of the tested LILRBA family members or to LILRB3, LILRB4 or LILRB5, as seen for the other clones (see Figure 5).

### LPS-driven cytokine secretion

To evaluate the effect of the LILRB2-BDs of the invention on the cytokine secretion levels of human monocyte-derived macrophages (HMDM), an LPS-driven cytokine secretion assay was performed as described in the following.

HMDMs were generated by culturing CD14+ cells for 6-8 days in complete medium (RPMI 1640, 10 % heat-inactivated bovine serum, Sigma Aldrich) in the presence of 50 ng/ml human macrophage colony stimulating factor (M-CSF) (Peprotech) at 37°C and 5 % CO2. Medium was exchanged and M-CSF replenished on day 3 of the culture. 50 000 - 100 000 HMDM cells were seeded in a flat-bottom 96-well plate and exposed to 100 ng/mL LPS in the presence of a serial dilution of PRO3744, PRO3696, and PRO3697. After 24 h of incubation at 37°C and 5 % CO₂, cell culture supernatants were harvested and human interleukin 1β (IL-1β), human interleukin 6 (IL-6) and tumor necrosis cell factor alpha (TNF-α) levels were quantified using the cytometric bead array method (CBA) from LegendPlex kit provided by Biolegend according to kit instructions (cat no.: 740930). Cytokine concentrations were interpolated from a standard curve generated for each cytokine and plotted against control conditions for calculation of EC₅₀ values. Data was acquired on Attune NxT Flow Cytometer (Thermo Fisher Scientific) and NovoCyte Quanteon 4025 Flow Cytometer (Agilent) and analyzed by Biolgend's LegendPLEX Data Analysis Software.

As shown in Table 10, IL-1β, IL-6 and TNF-α levels increased in the dose-dependent manner, indicating the activation of HMDM cells by PRO3744, PRO3696, and PRO3697 in the presence of LPS.

### Re-polarization of M2 macrophages

Further, to evaluate the potency of the LILRB2-BDs of the invention to stimulate re-polarization of M2 macrophages to their M1 state, a re-polarization assay has been performed, which follows the level of several surface markers that are indicative for re-polarization, such as CD86 (marker for M1 state) and CD163 and CD206 (markers for M2 state). The re-polarization assay was performed as follows.

Human peripheral blood mononuclear cells (PBMCs) were isolated from buffy coats of healthy donors by density gradient centrifugation (STEMCELL Technologies). CD14+ cells were isolated from PBMCs by positive selection with magnetic beads (Miltenyi Biotech, 130-050-201). HMDMs were generated by culturing CD14+ cells for 6-8 days in complete medium (RPMI 1640, 10 % heat-inactivated bovine serum, Sigma Aldrich) in the presence of 50 ng/ml human macrophage colony stimulating factor (M-CSF) (Peprotech) at 37°C and 5 % CO₂. Medium was exchanged and M-CSF replenished on day 3 of the culture.

HMDM cells were exposed to 25 ng/mL IL-4 in the presence of a serial dilution of PRO3744, PRO3696, and PRO3697 at 37°C and 5 % CO₂. After 24 h, flow cytometry assessment for the surface markers CD86, CD163 and CD206 was performed.

HMDMs were harvested from culturing flasks on day 6-8 using Accutase (Biowest), seeded at 50'000 cells per well in 96-well flat bottom plates (TPP) and treated with 25 ng/mL IL-4 (Peprotech, 200-04 - 50 µg) together with a serial dilution of test compounds or isotype antibodies (dose range 100 nM - 0.0006 nM) for 24 h at 37°C and 5 % CO2. After 24 h, cells were stained with Zombie Aqua Fixable Viability Kit (BioLegend) for 30 min at 4°C. Cells were incubated with the Fc receptor block (Human TruStain FcX, BioLegend, 422302) for 10 min at room temperature, to prevent nonspecific binding of the staining antibodies. Staining antibodies (all from BioLegend) were added to the cells for 30 min at 4°C: CD163-APC (cat. no. 333610), CD206-BV711 (cat. no. 321136), CD86-AF488 (cat. no. 305414), CD80-PE (cat. no. 305208) and CD14-PerCp-Cy5.5 (cat. no. 325622). Following washing step to remove primary antibodies, samples were fixed with a BD CellFix kit (BD, 340181): Cells were incubated in 50 µL of fixing solution for 20-30 min at 4°C in the dark. Samples were washed, resuspended in PBS and acquired with Attune NxT Flow Cytometer (Thermo Fisher Scientific). Data was analysed with the FlowJo software (Tree Star).

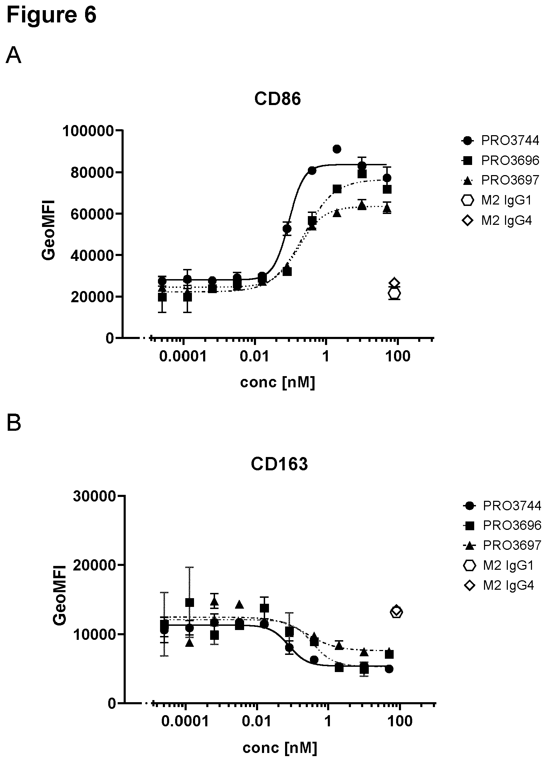

Figure 6 and Table 10 summarize the results obtained for CD86, CD163 and CD206 levels, *i. e*. markers associated with M1 (pro-inflammatory) and M2 (anti-inflammatory) macrophage polarization, in the presence of the anti-LILRB2 scFvs PRO3744, PRO3696, and PRO3697. For all anti-LILRB2-BDs, a dose dependent increase in the M1 marker CD86 and dose dependent decrease in the M2 markers CD163 and CD206 could be observed. These results indicate a shift of the macrophages towards M1 state (re-polarization), potentially suggesting an enhanced immune response.

**Table 10: LPS-driven cytokine secretion and repolarization of M2 macrophages**

| | **LPS-driven cytokine secretion** | | | **Re-polarization** | | |
|---|---|---|---|---|---|---|
| **PRO ID** | **IL-1β EC₅₀ [nM]** | **TNF-α EC₅₀ [nM]** | **IL-6 EC₅₀ [nM]** | **CD86 EC₅₀ [nM]** | **CD163 EC₅₀ [nM]** | **CD206 EC₅₀ [nM]** |
| PRO3744 | 0.7788 | 1.1031 | 0.8035 | 0.1171 | 0.1070 | 0.1361 |
| PRO3696 | 0.4462 | 0.8912 | 0.8036 | 0.2433 | 0.3715 | 0.5867 |
| PRO3697 | 0.2788 | 2.2044 | 0.4500 | 0.2140 | 0.1726 | 0.5117 |

### 1.8 Biophysical characterization of suitable anti-LILRB2 binding domains (scFv format)

### Storage stability study

Humanized scFvs were subjected to stability studies, such as a four-week stability study, in which the scFvs were formulated in an aqueous buffer (50 mM NaCiP, 150 mM NaCl, pH 6.4) at 10 mg/ml and stored at < -80°C, 4°C and 40°C for four weeks. At the minimum, the fractions of monomers and oligomers in the formulation were evaluated by integration of SE-HPLC peak areas after two weeks and at the end of each study. Additional time points were recorded for most of the molecules. Table 11 (monomer content) and Table 12 (protein content) compare day 14 (d14) and endpoint measurements obtained on day 28 (d28) of the study.

Most molecules exhibited >5 % monomer loss upon storage for 28 days at 4°C. None of the scFvs exhibited considerable protein content loss at any tested storage temperature and time point. Most pronounced differences in monomeric content between molecules were observed in samples stored for 14/28 days at 40°C.

### Thermal unfolding

Thermal stability of scFvs was analyzed by nano dynamic scanning fluorimetry (nDSF) using a NanoTemper, allowing the determination of the onset of unfolding (Tonset), midpoint of unfolding (Tm). Tm and Tonset results of duplicate/triplicate measurements are summarized in Table 13. As can be deduced from Table 13, all molecules exhibit high melting temperatures of at least 59°C, most of the molecules exhibit melting temperatures of >60°C or >70°C.

**Table 11: Course of monomeric content and monomeric content loss upon sample storage at a concentration of 10 g/L and temperatures of -80°C, 4°C and 40°C for up to 28 days.**

| **PRO ID** | **Description** | **Day 0** | **Day 14 (MC -80°C)** | **Day 28 (MC -80°C)** | **Day 14 (MC 4°C)** | **Day 28 (MC 4°C)** | **Day 14 (MC 40°C)** | **Day 28 (MC 40°C)** | **Day 14 (MC% -80°C)** | **Day 28 (MC% -80°C)** | **Day 14 (MC% 4°C)** | **Day 28 (MC% 4°C)** | **Day 14 (MC% 40°C)** | **Day 28 (MC% 40°C)** |
|---|---|---|---|---|---|---|---|---|---|---|---|---|---|---|
| PRO3744 | 68-18-E11-sc07 | 98.9 | n.a. | 99.9 | 99.9 | 99.9 | 99.8 | 98.5 | n.a. | -1.0 | -1.0 | -1.0 | -0.9 | 0.4 |
| PRO3697 | 69-06-G04-sc05 | 98.1 | n.a. | 97.1 | 93.2 | 92.2 | 92.3 | 91.8 | n.a. | 1.0 | 5.0 | 6.0 | 5.9 | 6.5 |
| PRO3696 | 69-01-E02-sc06 | 99.8 | n.a. | 99.0 | 98.0 | 98.9 | 97.6 | 95.9 | n.a. | 0.9 | 1.8 | 0.9 | 2.2 | 3.9 |

**Table 12: Course of protein content and protein content loss upon sample storage at a concentration of 10 g/L and temperatures of -80°C, 4°C and 40°C for up to 28 days.**

| **PRO ID** | **Description** | **Day 0 (PC)** | **Day 14 (PC -80°C)** | **Day 28 (PC -80°C)** | **Day 14 (PC 4°C)** | **Day 28 (PC 4°C)** | **Day 14 (PC 40°C)** | **Day 28 (PC 40°C)** | **Day 14 (PC% -80°C)** | **Day 28 (PC% -80°C)** | **Day 14 (PC% 4°C)** | **Day 28 (PC% 4°C)** | **Day 14 (PC% 40°C)** | **Day 28 (PC% 40°C)** |
|---|---|---|---|---|---|---|---|---|---|---|---|---|---|---|
| PRO3744 | 68-18-E11-sc07 | 9.9 | n.a. | 9.9 | 9.8 | 9.9 | 10.1 | 10.2 | n.a. | 0.3 | 0.9 | 0.4 | -1.7 | -3.5 |
| PRO3697 | 69-06-G04-sc05 | 10.5 | n.a. | 10.7 | 10.3 | 10.7 | 10.5 | 11.0 | n.a. | -1.8 | 1.4 | -1.9 | -0.1 | -4.5 |
| PRO3696 | 69-01-E02-sc06 | 10.5 | n.a. | 11.0 | 10.6 | 10.6 | 10.8 | 10.8 | n.a. | -4.4 | -1.2 | -0.9 | -2.4 | -2.7 |

**Table 13: nDSF measurement of scFvs using NanoTemper device for determination of Tm and Tonset.**

| **PRO ID** | **Description** | **Tₒₙₛₑₜ [°C]** | **Tₘ [°C]** |
|---|---|---|---|
| PRO3744 | 68-18-E11-sc07 | 64.1 | 74.6 |
| PRO3697 | 69-06-G04-sc05 | 57.9 | 65.5 |
| PRO3696 | 69-01-E02-sc06 | 53.2 | 61.1 |

| | | | |
|---|---|---|---|
| PC: protein content [mg/mL] MC: monomer content [%] | | | |

### Example 2: Anti-CD47 x PDL1x LILRB2 trispecific antibodies

### 2.1 Manufacture of anti-CD47 x PDL1x LILRB2 trispecific antibodies

The trispecific antibodies according to the present invention have been produced analogous to the methods described in Example 1.6. Briefly, expression of multispecific constructs was performed in ExpiCHO-S cells using the transient ExpiCHO expression system (Gibco). Expression cultures were cultivated in batch using shaking flasks for 8 days at 37°C. The culture supernatants were separated from cells by centrifugation followed by a 0.22 µm sterile filtration. All target proteins were captured from the clarified culture supernatants by protein A affinity chromatography and were polished by a size-exclusion chromatography (SEC) step. For the quality control of the manufactured material, standard analytical methods such as SE-HPLC, SDS-PAGE, and UV280 were used. Table 14 shows a production overview of these molecules. All molecules were finalized to a monomeric content ≥95 % after polishing or dialysis, respectively.

**Table 14: Description of produced anti-CD47 x PDL1x LILRB2 trispecific antibodies**

| | **Identifier** | **PD-L1 binder** | **CD47 binder** | **LILRB2 binder** | **Post-capture titer [mg/L]*** | **Post-SEC monomeric purity [%]** |
|---|---|---|---|---|---|---|
| **anti-PDL1 x CD47 x LILRB2** | | | | | | |
| | **PRO5052** | 33-03-G02-sc24 | 70-26-B04-sc03 | 68-18-E11-sc07 | 290 | 97.7 |
| | **PRO5055** | 33-03-G02-sc24 | 70-26-B04-sc03 | 68-18-E11-sc07 | 143 | 97.6 |
| | **PRO4672** | 37-20-B03-sc18 | 70-26-B04-sc03 | 68-18-E11-sc07 | 278 | 99.1 |
| | **PRO4687** | 37-20-B03-sc18 | 70-26-B04-sc03 | 68-18-E11-sc07 | 148 | 99.2 |

### 2.2 Biophysical characterization of anti-CD47 x PDL1x LILRB2 trispecific antibodies according to the invention

### Storage stability study

PRO4672, PRO5052, PRO4687 and PRO5055 were subjected to stability studies such as a four-week stability study, in which the scFvs were formulated in an aqueous buffer (20 mM histidine, 125 mM NaCl, 0.02 % Poloxamer 188, pH 6.0) at 10 mg/ml and stored at 4°C, 25°C and 40°C for 2 weeks, 4 weeks and up to 8 weeks. At the minimum, the fractions of monomers and oligomers in the formulation were evaluated by integration of SE-HPLC peak areas after two weeks, four weeks and at the end of each study. Table 15 summarizes stability results obtained at t2w (2 weeks), t4w (4 weeks) and endpoint measurements obtained at t8w (8 weeks) of the study.

As shown in Table 15, PRO4672, PRO5052, PRO4687 and PRO5055 showed good storage stability at 4°C, 25°C and 40°C. The loss of monomeric content after storage at 25°C for 4 weeks or at 4°C for 8 weeks was less than 1 %. More surprisingly, the loss of monomeric content after storage at 40°C for 4 weeks was still less than 5 %. None of the stable molecules showed considerable loss of protein content at any temperature.

### Freeze-thaw stability

As in the early development stages the drug substance is usually stored frozen, formulation and fill/finish processing will require some freezing and thawing operations. The compatibility of the top performing trispecific molecules with respect to freeze-thawing (F/T) cycles (colloidal stability) was assessed.

For the F/T stability assessment the same analytical methods (SE-HPLC, cGE) and parameters (% monomer content and % monomer loss) as for the storage stability study were applied to monitor the quality of the molecules over five F/T cycles. Table 15 illustrates that after five F/T cycles (F/T) the loss of monomer content was less than 1 %. None of the stable molecules showed considerable loss of protein content at any temperature (data not shown).

**Table 15: Stability studies**

| PRO-ID | t0 | Monomer content by SE-HPLC [rel. %] | | | | | | Δ Monomer content by SE-HPLC [rel. %] | | | | | | nDSF | | |
|---|---|---|---|---|---|---|---|---|---|---|---|---|---|---|---|---|
| | | F/T | 40°C | | 25°C | 4°C | | F/T | 40°C | | 25°C | 4°C | | Ton | Tm1 | Tm2 |
| | | 5 | t2w | t4w | t4w | t4w | t8w | 5 | t2w | t4w | t4w | t4w | t8w | [°C] | [°C] | [°C] |
| PRO4672 | 98.5 | 98.6 | 98.3 | 97.4 | 98.9 | 98.9 | 98.4 | 0.1 | -0.2 | -1.1 | 0.4 | 0.4 | -0.1 | 52.3 | 59.9 | 72.3 |
| PRO5052 | 98.9 | 98.6 | 97.9 | 96.5 | 98.5 | 98.7 | 98.3 | -0.3 | -1.0 | -2.4 | -0.4 | -0.2 | -0.6 | 56.5 | 60.3 | 67.8 |
| PRO4687 | 98.5 | 98.1 | 96.3 | 94.0 | 97.9 | 98.3 | 98.0 | -0.4 | -2.2 | -4.4 | -0.6 | -0.2 | -0.5 | 55.4 | 60.3 | 70.6 |
| PRO5055 | 99.2 | 98.9 | 97.9 | 97.0 | 98.8 | 98.9 | 99.0 | -0.3 | -1.3 | -2.2 | -0.4 | -0.3 | -0.2 | 57.4 | 60.4 | 70.6 |

### 2.3 Pharmacodynamic characterization of anti-CD47 x PDL1 x LILRB2 trispecific antibodies according to the invention

### Binding affinity to human CD47, PDL1 and LILRB2

Affinity of trispecific molecules to human and cynomolgus CD47, and human and cynomolgus PDL1 was determined by SPR analysis as described in Example 1.7. For affinity measurements to human LILRB2, a different SPR measurement procedure was applied to determine the binding affinity without avidity effects.

Briefly, binding affinity without avidity was determined by using a proprietary anti-framework rabbit IgG (rlgG21-57-20-B11) immobilized to a carboxymethylated dextran surface (CM5 sensorchip; Biacore, Cytiva) to capture the multispecific antibodies according to the present invention on the surface. For each antigen, a titration series of the respective antigen was injected as analyte. After each capture-analyte injection cycle, every flow channel on the sensor chip was regenerated (3 M MgCl₂), and a multispecific antibody according to the present invention was captured again followed by a different concentration of the antigen. The binding kinetics to the human antigens were measured using a multicycle kinetic assay, with eleven analyte concentrations ranging from 0.088 to 90 nM, 1:2 diluted in running buffer (HEPES, 150 mM NaCl, 0.05 % Tween-20, pH 7.5; Bioconcept).

The results are shown in Table 16.

**Table 16: Affinity of different multispecific test constructs to human and cynomolgus CD47, human and cynomolgus PDL1, and human and cynomolgus LILRB2.**

| **Protein ID** | **Target** | **Affinity** kₐ (M⁻¹ s⁻¹) | k_{d} (s⁻¹) | K_{D} (M) |
|---|---|---|---|---|
| PRO4672 | human CD47 | 4.48E+05 | 2.56E-02 | 5.72E-08 |
| | human PDL1 | 1.05E+06 | 1.26E-04 | 1.20E-10 |
| | human LILRB2 *⁾ | 2.4E+05 | 8.9E-05 | 3.7E-10 |
| PRO4687 | human CD47 | 8.11E+05 | 2.04E-02 | 2.52E-08 |
| | human PDL1 | 1.31E+06 | 1.16E-04 | 8.85E-11 |
| | human LILRB2 *⁾ | 2.6E+05 | 9.3E-05 | 3.6E-10 |
| PRO5052 | human CD47 | 6.6E+05 | 2.2E-02 | 3.4 ±0.2E-08 |
| | cynom. CD47 | | | 3.2 ±0.1E-08 |
| | human PDL1 | 2.3E+06 | 2.9E-05 | 1.3 ±0.4E-11 |
| | cynom. PDL1 | | | 1.2 ±0.2E-11 |
| | human LILRB2 *⁾ | 2.9E+05 | 5.4E-05 | 1.9 ±0.8E-10 |
| | cynom. LILRB2 *⁾ | | | no binding |
| PRO5055 | human CD47 | 5.1E+05 | 1.1E-02 | 2.1 ±0.04E-08 |
| | cynom. CD47 | | | 2.1 ±0.1E-08 |
| | human PDL1 | 2.2E+06 | 3.3E-05 | 1.6 ±0.7E-11 |
| | cynom. PDL1 | | | 8.4 ±0.9E-12 |
| | human LILRB2 *⁾ | 2.6E+05 | 9.2E-05 | 3.5E-10 |
| | cynom. LILRB2 *⁾ | | | no binding |

| | | | | |
|---|---|---|---|---|
| *) Affinity to human and cynomolgus LILRB2 has been measured without avidity. | | | | |

### Re-polarization of M2 macrophages and LPS-driven cytokine secretion

The ability of the anti-CD47 x PDL1 x LILRB2 antibodies to re-polarize M2 macrophages and to foster LPS-driven cytokine secretion was assessed using the methods disclosed in Example 1.7.

It was observed that the anti-CD47 x PDL1 x LILRB2 antibodies of the invention could inhibit LILRB2, causing a dosage-dependent rise in the M1 marker CD86 and a decline in the M2 markers CD163 and CD206, with a similar potency to that of the monospecific anti-LILRB2 IgG4 PRO4710 (see Table 17). On the other hand, the LILRB2-BD-devoid trispecific variants PRO5053 and PRO5056 (HSA-BD as LILRB2 mock binding domain), tested at a 50 nM dose, showed no re-polarization activity.

**Table 17: Re-polarization potency of M2 macrophages for the anti-LILRB2 IgG PRO4710 and the trispecific antibodies PRO4672, PRO4687, PRO5052 and PRO5055**

| **PRO ID** | **CD86 EC₅₀ [nM]** | **CD163 EC₅₀ [nM]** | **CD206 EC₅₀ [nM]** |
|---|---|---|---|
| **PRO4710** | 0.119 ± 0.070 | 0.072 ± 0.008 | 0.203 ± 0.129 |
| **PRO4672** | 0.079 ± 0.009 | 0.067 ± 0.004 | 0.120 ± 0.043 |
| **PRO4687** | 0.078 ± 0.009 | 0.070 ± 0.009 | 0.124 ± 0.077 |
| **PRO5052** | 0.082 ± 0.006 | 0.073 ± 0.001 | 0.134 ± 0.082 |
| **PRO5055** | 0.085 ± 0.012 | 0.058 ± 0.003 | 0.128 ± 0.071 |

The trispecific antibodies according to the invention further exhibit the ability to foster LPS-driven cytokine secretion (see Table 18), due to the presence of the LILRB2 arm, which is confirmed through comparison with the respective mock compounds PRO5053 and PRO5056 (hSA-BD as LILRB2 mock binding domain), for which no activity could be observed.

**Table 18: Induction of cytokine secretion for the anti-LILRB2 IgG PRO4710, the trispecific antibodies PRO4672, PRO4687, PRO5052, PRO5053, PRO5055 and PRO5056, and reference compounds magrolimab, atezolizumab, and PRO3599**

| **PRO ID** | **IL-1β EC₅₀ [nM]** | **TNF-α EC₅₀ [nM]** | **IL-6 EC₅₀ [nM]** |
|---|---|---|---|
| **PRO4710** | 0.248 ± 0.136 | 0.169 ± 0.026 | 0.153 ± 0.043 |
| **PRO4672** | 0.096 ± 0.026 | 0.123 ± 0.037 | 0.078 ± 0.007 |
| **PRO4687** | 0.205 ± 0.166 | 0.175 ± 0.038 | 0.097 ± 0.020 |
| **PRO5052** | 0.082 ± 0.028 | 0.117 ± 0.052 | 0.077 ± 0.024 |
| **PRO5053** | n.a. | n.a. | n.a. |
| **PRO5055** | 0.0705 | 0.141 ± 0.066 | 0.063 ± 0.019 |
| **PRO5056** | n.a. | n.a. | n.a. |

Both assays demonstrate the efficacy of LILRB2 inhibition.

### 2.4 Induction of T cell proliferation

To evaluate whether the trispecific antibodies of the invention are capable to activate T cell proliferation, these antibodies were tested in an immunosuppressive assay. The immunosuppressive assay used aims to track recovery of T cell proliferation in the co-culture with suppressive macrophages in the presence of T cell stimulation and test compounds. The assay was performed as follows.

T cells were isolated from PBMC fraction with a magnetic Pan T Cell negative isolation kit (Miltenyi Biotec, cat no. 130-096-535). Bulk T cell population was used containing CD4+ and CD8+ T cells. T cells were stained with a cell proliferation tracking dye (CTV CellTrace dye, Thermo Fisher Scientific, cat no. C34557) by incubation of T cells with the CTV dye (5 µM working solution) for 20 min at 37°C. To stop the labelling reaction, 5x volume of complete medium containing 10 % FBS was added for 5 min at room temperature followed by extensive washing.

T cells were stimulated to proliferate by a co-culture in 96 flat-bottom well plates (TPP) with coated anti-CD3 (at 2 ug/mL, BioLegend, cat no. 317326) and soluble anti-CD28 mAbs (4 µg/mL, BioLegend, cat no. 302934). Suppressive human monocyte-derived macrophages (HMDM) were generated by treatment with IL-10 (10 ng/mL, BioLegend cat no. 571004,) and TGF-β (20 ng/mL, Peprotech, cat no. 100-21) for 24 h at 5 % CO₂ and 37°C prior to co-culture with T cells. HMDM were added to the culture in different ratios, e.g. 1:1, 2.5:1 and 5:1 (T cell : HMDM). Total number of cells varied from 120 k to 150 k per well. Co-culture of T cells and macrophages lasted for 5 days at 37°C and 5 % CO₂. As a positive control, stimulated T cells cultured without HMDM were used (max. proliferation observed). As a negative control CTV-labelled, unstimulated T cells were used (population showed no proliferation).

T cell proliferation was measured by quantification of CTV dilution, which increases with every cell division. Data was collected with flow cytometry method, where T cells and HMDM were identified by staining with mAb (all from BioLegend: CD14-APC cat. no. 325608, CD4-FITC 317408, CD8 PercP cat no. 344708). Live/dead stain (Zombie NIR Fixable Viability Kit, BioLegend, cat. No. 423106 ) was employed to exclude dead cells from subsequent analysis. Data was acquired with Attune NxT Flow Cytometer (Thermo Fisher Scientific) and analysed with the FlowJo software (Tree Star) and T cell proliferation was quantified by calculating the proliferation index, which is the total number of divisions divided by the number of cells that went into division.

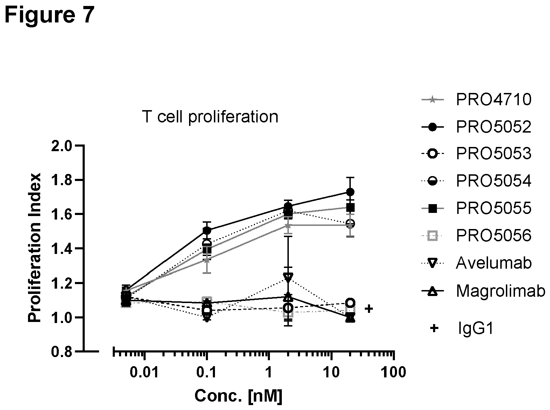

Trispecific molecules of the invention including PRO5056 having a mock CD47-BD (hSA-BD as CD47 mock binding domain) demonstrate a dose-dependent induction of T-cell proliferation, as indicated by the significant CTV dye dilution and proliferation index (see Figure 7). This heightened activity is attributed to the inhibition of LILRB2, as evidenced by the singular effectiveness of the IgG compound as a standalone agent. Magrolimab, avelumab and atezolizumab IgGs showed no significant impact after treatment.

The induction of T cell proliferation is specifically propelled by the inhibition of LILRB2, with no discernible involvement of the PDL1- and CD47-BDs in this assay. Additionally, the trispecific antibodies of the invention exhibit dose-dependent activity in this context.

### 2.5 LILRB2 selectivity assessment

To assess whether the trispecific antibodies according to the invention selectively bind to LILRB2 and not to other members of protein family LILRA and LILRB, a cellular ELISA (CELISA) using transiently transfected CHO cells was developed. The method applied for this assessment was the same as described in Example 1.7.

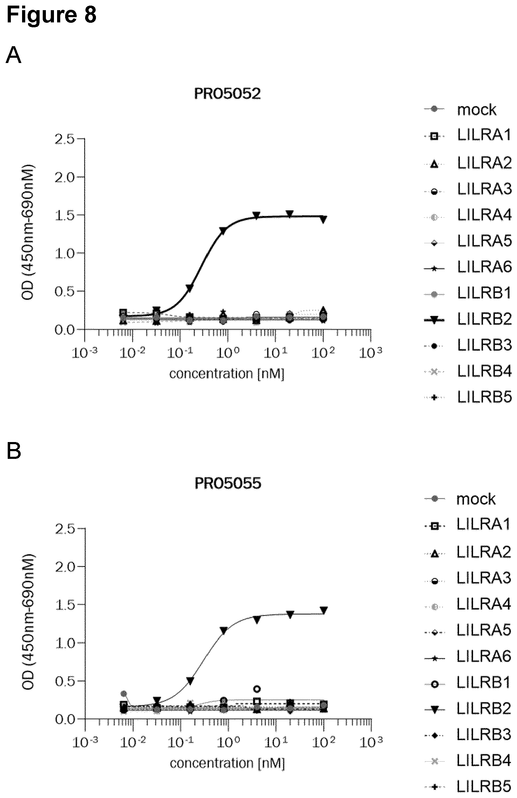

As shown in Figure 8, trispecific antibodies PRO5052 and PRO5055 demonstrated potent and selective binding to LILRB2.

## Claims

1. An antibody binding domain, which specifically binds to LILRB2 (LILRB2-BD), comprising:
a) a variable heavy chain (VH),
wherein the variable heavy chain comprises, from N-terminus to C-terminus, the regions HFR1-HCDR1-HFR2-HCDR2-HFR3-HCDR3-HFR4, wherein each HFR designates a heavy chain framework region, and each HCDR designates a heavy chain complementarity-determining region, and
b) a variable light chain (VL),
wherein the variable light chain comprises, from N-terminus to C-terminus, the regions LFR1-LCDR1-LFR2-LCDR2-LFR3-LCDR3-LFR4, wherein each LFR designates a light chain framework region, and each LCDR designates a light chain complementarity-determining region,
wherein
said HCDR1 has the sequence of SEQ ID NO: 1,
said HCDR2 has the sequence of SEQ ID NO: 2,
said HCDR3 has the sequence of SEQ ID NO: 3,
said LCDR1 has the sequence of SEQ ID NO: 4,
said LCDR2 has the sequence of SEQ ID NO: 6, and
said LCDR3 has the sequence of SEQ ID NO: 7;
or wherein
said HCDR1 has the sequence of SEQ ID NO: 1,
said HCDR2 has the sequence of SEQ ID NO: 2,
said HCDR3 has the sequence of SEQ ID NO: 3,
said LCDR1 has the sequence of SEQ ID NO: 4,
said LCDR2 has the sequence of SEQ ID NO: 5, and
said LCDR3 has the sequence of SEQ ID NO: 7;
or wherein
said HCDR1 has the sequence of SEQ ID NO: 20,
said HCDR2 has the sequence of SEQ ID NO: 21,
said HCDR3 has the sequence of SEQ ID NO: 22,
said LCDR1 has the sequence of SEQ ID NO: 23,
said LCDR2 has the sequence of SEQ ID NO: 24, and
said LCDR3 has the sequence of SEQ ID NO: 26;
or wherein
said HCDR1 has the sequence of SEQ ID NO: 20,
said HCDR2 has the sequence of SEQ ID NO: 21,
said HCDR3 has the sequence of SEQ ID NO: 22,
said LCDR1 has the sequence of SEQ ID NO: 23,
said LCDR2 has the sequence of SEQ ID NO: 25, and
said LCDR3 has the sequence of SEQ ID NO: 26;
or wherein
said HCDR1 has the sequence of SEQ ID NO: 36,
said HCDR2 has the sequence of SEQ ID NO: 37,
said HCDR3 has the sequence of SEQ ID NO: 38,
said LCDR1 has the sequence of SEQ ID NO: 39,
said LCDR2 has the sequence of SEQ ID NO: 40, and
said LCDR3 has the sequence of SEQ ID NO: 42;
or wherein
said HCDR1 has the sequence of SEQ ID NO: 36,
said HCDR2 has the sequence of SEQ ID NO: 37,
said HCDR3 has the sequence of SEQ ID NO: 38,
said LCDR1 has the sequence of SEQ ID NO: 39,
said LCDR2 has the sequence of SEQ ID NO: 41, and
said LCDR3 has the sequence of SEQ ID NO: 42.

2. The antibody binding domain of claim 1, wherein said LILRB2-BD:
- is in a format selected from a Fab, an scFab, an Fv, a dsFv, an scFv and a dsscFv, particularly from a Fab, Fv and scFv; or
- is in a format selected from an Fv, a dsFv, an scFv and a dsscFv, particularly from an Fv and scFv.

3. The antibody binding domain of claim 1 or 2, wherein
a) said LFR1, LFR2, LFR3 and LFR4 are selected from Vκ subtypes, particularly from the Vκ1 and Vκ3 subtypes, particularly are of the Vκ1 subtype; or
b) said LFR1, LFR2 and LFR3 are selected from Vκ subtypes, particularly from the Vκ1 and Vκ3 subtypes, particularly are of the Vκ1 subtype, and said LFR4 is selected from a Vλ framework 4, particularly is a Vλ framework 4 comprising an amino acid sequence having at least 80, 90 percent identity to any of SEQ ID NOs: 62 to 70, more particularly a Vλ framework 4 selected from any of SEQ ID NOs: 62 to 70, particularly has a Vλ framework 4 according to SEQ ID NO: 62 or 70.

4. The antibody binding domain of any one of the claims 1 to 3, comprising:
a) a VH sequence at least 90, 91, 92, 93, 94, 95, 96, 97, 98 or 99 percent identical to the amino acid sequence of SEQ ID NO: 8; and
a VL sequence at least 90, 91, 92, 93, 94, 95, 96, 97, 98 or 99 percent identical to any one of the amino acid sequences selected from SEQ ID NOs: 12, 14 and 15, particularly selected from SEQ ID NOs: 12 and 15, particularly to SEQ ID NO: 15; or
b) a VH sequence at least 90, 91, 92, 93, 94, 95, 96, 97, 98 or 99 percent identical to the amino acid sequence of SEQ ID NO: 9; and
a VL sequence at least 90, 91, 92, 93, 94, 95, 96, 97, 98 or 99 percent identical to any one of the amino acid sequences selected from SEQ ID NOs: 11 and 12, particularly to SEQ ID NO: 12; or
c) a VH sequence at least 90, 91, 92, 93, 94, 95, 96, 97, 98 or 99 percent identical to the amino acid sequence of SEQ ID NO: 10; and
a VL sequence at least 90, 91, 92, 93, 94, 95, 96, 97, 98 or 99 percent identical to the amino acid sequence of SEQ ID NO: 13; or
d) a VH sequence at least 90, 91, 92, 93, 94, 95, 96, 97, 98 or 99 percent identical to the amino acid sequence of SEQ ID NO: 27; and
a VL sequence at least 90, 91, 92, 93, 94, 95, 96, 97, 98 or 99 percent identical to any one of the amino acid sequences selected from SEQ ID NOs: 30, 31 and 32, particularly selected from SEQ ID NOs: 30 and 32, particularly to SEQ ID NO: 32; or
e) a VH sequence at least 90, 91, 92, 93, 94, 95, 96, 97, 98 or 99 percent identical to the amino acid sequence of SEQ ID NO: 28; and
a VL sequence at least 90, 91, 92, 93, 94, 95, 96, 97, 98 or 99 percent identical to any one of the amino acid sequences selected from SEQ ID NOs: 29 and 30, particularly to SEQ ID NO: 30; or
f) a VH sequence at least 90, 91, 92, 93, 94, 95, 96, 97, 98 or 99 percent identical to the amino acid sequence of SEQ ID NO: 43; and
a VL sequence at least 90, 91, 92, 93, 94, 95, 96, 97, 98 or 99 percent identical to any one of the amino acid sequences selected from SEQ ID NOs: 46, 47 and 48, particularly selected from SEQ ID NOs: 46 and 48, particularly to SEQ ID NO: 48; or
g) a VH sequence at least 90, 91, 92, 93, 94, 95, 96, 97, 98 or 99 percent identical to the amino acid sequence of SEQ ID NO: 44; and
a VL sequence at least 90, 91, 92, 93, 94, 95, 96, 97, 98 or 99 percent identical to the amino acid sequence of SEQ ID NO: 46.

5. The antibody binding domain of any one of the preceding claims, which is an scFv antibody fragments having a sequence selected from SEQ ID NOs: 16, 17, 18, 19, 33, 34, 35, 49, 50 and 51.

6. An monospecific antibody comprising two antibody binding domains according to any one of the claims 1 to 5.

7. The antibody of claim 6, which has amino acid sequences selected from:
- SEQ ID NO: 75 and SEQ ID NO: 76 (PRO3603);
- SEQ ID NO: 77 and SEQ ID NO: 78 (PRO3635); and
- SEQ ID NO: 79 and SEQ ID NO: 80 (PRO4710).

8. A multispecific antibody comprising:
a) one or two antibody variable domains as defined in any one of claims 1 to 5;
b) at least one binding domain, which specifically binds to a target different from LILRB2.

9. The multispecific antibody of claim 8, wherein the multispecific antibody comprises an immunoglobulin Fc region.

10. The multispecific antibody of claim 9, wherein the immunoglobulin Fc region has one or more amino acid modifications selected from the group consisting of:
a) modifications to enhance heterodimerization of heavy chains, such as the knob-into-hole (KiH) technology;
b) modifications that increase or decrease Fc receptor binding, when compared to the respective unmodified immunoglobulin Fc region thereof;
c) modifications that increase serum half-life, when compared to the respective unmodified immunoglobulin Fc region thereof.

11. A nucleic acid or two nucleic acids encoding the antibody binding domain of any one of claims 1 to 5, or the antibody of claim 6 or 7; or a nucleic acid or two nucleic acids or three nucleic acids encoding the multispecific antibody of any one of the claims 8 to 10.

12. A vector or two vectors or three vectors comprising the nucleic acid or two nucleic acids or three nucleic acids of claim 11.

13. A host cell or host cells comprising the vector or the two vectors or the three vectors of claim 12.

14. A pharmaceutical composition comprising the antibody of claim 6 or 7, or the multispecific antibody of any one of the claims 8 to 10, and a pharmaceutically acceptable carrier.

15. The antibody of claim 6 or 7, or the multispecific antibody of any one of the claim 8 to 10, or the pharmaceutical composition of claim 14 for use in the treatment of a disease, particularly a human disease, particularly a human disease selected from cancer; more particular for use in the treatment of a cancer selected from:
a benign or especially malignant tumor, solid tumors, mesothelioma, brain cancer, kidney cancer, liver cancer, adrenal gland cancer, bladder cancer, breast cancer, stomach cancer (e. g., gastric tumors), esophageal cancer, ovarian cancer, cervical cancer, thymic cancer, choriocarcinoma, oral cancer, salivary cancer, colon cancer, rectum cancer, prostate cancer, pancreatic cancer, lung cancer (e. g., non-small cell lung cancer and small cell lung cancer), vaginal cancer, thyroid cancer, melanoma (e. g., unresectable or metastatic melanoma), renal cell carcinoma, sarcoma, glioma, glioblastoma, multiple myeloma or gastrointestinal cancer, especially colon carcinoma or colorectal adenoma, a tumor of the neck and head, endometrial cancer, Cowden syndrome, Lhermitte-Duclos disease, Bannayan-Zonana syndrome, prostate hyperplasia, a neoplasia, especially of epithelial character, preferably mammary carcinoma or squamous cell carcinoma, chronic lymphocytic leukemia, chronic myelogenous leukemia (e. g., Philadelphia chromosome-positive chronic myelogenous leukemia), acute lymphoblastic leukemia (e. g., Philadelphia chromosome-positive acute lymphoblastic leukemia), non-Hodgkin's lymphoma, plasma cell myeloma, Hodgkin's lymphoma, a leukemia, and any combination thereof.
